# EUROPÄISCHE PATENTANMELDUNG

(11) **EP 1 803 723 A1**
(43) Veröffentlichungstag der Anmeldung: **04.07.2007**
(21) Anmeldenummer: 06090001.6
(22) Anmeldetag: 03.01.2006
(51) Int. Cl.: C07D 487/08, A61P 35/00

(54) **(2,4,9-TRIAZA-1(2,4)-PYRIMIDINA-3(1,3)-BENZENACYCLONONAPHAN-3^4-YL)-SULFOXIMID DERIVATE ALS SELEKTIVE INHIBITOREN DER AURORA KINASE ZUR BEHANDLUNG VON KREBS**

(71) Anmelder: Bayer Schering Pharma Aktiengesellschaft, 13353 Berlin (DE)
(72) Erfinder: Lücking, Ulrich, 10245 Berlin (DE); Bader, Benjamin, 10245 Berlin (DE); Siemeister, Gerhard, 13503 Berlin (DE)

(57) **Zusammenfassung**

Die Erfindung betrifft makrozyklische Anilino-Pyrimidine mit substituiertem Sulfoximin der allgemeinen Formel I, Verfahren zu deren Herstellung, sowie deren Verwendung als Arzneimittel zur Behandlung von Krebs. 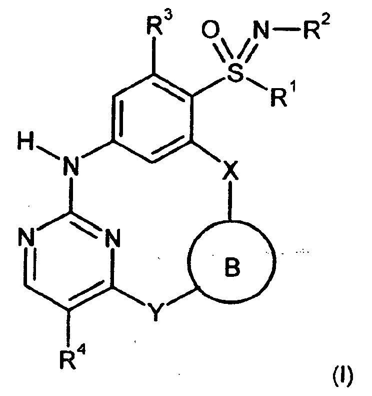 in der
B für eine Prop-1,3-ylen-, But-1,4-ylen-, Pent-1,5-ylen- oder Hex-1,6-ylen- gruppe, steht, die ein- oder mehrfach, gleich oder verschieden substituiert sein kann
R² für R⁵, -SO₂-R⁶, -C(O)O-R⁶, -C(O)-R⁶, -C(O)-NR¹¹R¹², -C(S)-NR¹¹R¹²,
-Si(R⁷R⁸R⁹), -R¹⁰-Si(R⁷R⁸R⁹) oder -SO₂-R¹⁰-Si(R⁷R⁸R⁹) steht,
X für -S-, -S(O)-, -NH- oder -O- steht,
Y für -NR¹³-, -S-, -S(O)-, oder -O- steht.
Die anderen Substituierten sind in den Ansprüchen spezifiziert.

Es wurde überraschend gefunden, dass die Substitution des Sulfoximin- Stickstoffs zu Verbindungen führt, die selektiv Zellzykluskinasen des Typs 2 inhibieren, insbesondere die Aurora inhibieren und gleichzeitig selektiv gegen Zellzykluskinasen des Typs 1, wie Zyklin-abhängige Kinasen, sind.

Die Substitution des Sulfoximinstickstoffatoms eröffnet weiterhin die Möglichkeit, Verbindungen zur Verfügung zu stellen, die neben der Inhibierung der Zellzykluskinasen des Typs 2 eine weitere Kinase inhibieren, so dass das Tumorwachstum effektiv gehemmt wird, insbesondere eine Kinase aus den Kinasefamilien der Rezeptortyrosinkinasen, der Kontrollpunktkinasen, der anti-apoptotischen Kinasen oder der migratorischen Kinasen.

## Beschreibung

Die Erfindung betrifft makrozyklische Anilino-Pyrimidine mit substituiertem Sulfoximin, Verfahren zu deren Herstellung, sowie deren Verwendung als Arzneimittel.

In eukaryontischen Zellen werden viele biologische Prozesse wie z.B. DNA-Replikation, Energiestoffwechsel, Zellwachstum oder -differenzierung durch reversible Phosphorylierung von Proteinen reguliert. Der Phosphorylierungsgrad eines Proteins hat unter anderem Einfluss auf die Funktion, Lokalisation oder Stabilität von Proteinen. Die Enzymfamilien der Proteinkinasen und Proteinphosphatasen sind verantwortlich für die Phosphorylierung bzw. Dephosphorylierung von Proteinen.

Die sequentielle Aktivität der Zellzykluskinasen aus den Familien der Zyklin-abhängigen Kinasen (cylin-dependent kinase, CDK), der Polo-like Kinasen (Plk) und der Aurora Kinasen steuert die Teilung und damit die Vermehrung einer Zelle. Daher sind diese Zellzykluskinasen besonders interessante Ziele für die Entwicklung kleiner inhibitorischer Moleküle, die zur Behandlung von Krebs oder anderen Erkrankungen, die Störungen der Zellproliferation zur Ursache haben, verwendet werden können.

Zellzykluskinasen können unterschieden werden hinsichtlich der durch sie regulierten Zellzyklusphase:
a) Zellzykluskinasen des Typs 1
   Unter den Zellzykluskinasen des des Typs 1 im Sinne der vorliegenden Erfindung sind alle Zellzykluskinasen zu verstehen, deren Aktivität nicht auf die Mitose beschränkt ist.
   Zu den Zellzykluskinasen des Typs 1 gehören im wesentlichen die Zyklin-abhängigen Kinasen (cyclin-dependent kinase: cdk) und die polo-like Kinasen (Plk).
b) Zellzykluskinasen des Typs 2
   Unter den Zellzykluskinasen des Typs 2 im Sinne der vorliegenden Erfindung sind alle Zellzykluskinasen zu verstehen, deren Aktivität im Zellzyklus auf die M-Phase (Mitose) beschränkt ist.
   Zu den Zellzykluskinasen des Typs 2 gehören im wesentlichen die Aurora Kinasen.

Die Inhibierung von Zellzykluskinasen des Typs 1, wie CDK oder Plk, schließt aus, die Tumorzelle in der empfindlicheren M-Phase zu treffen da sie bereits in einer früheren Phase des Zellzyklus arretiert wird.

Es besteht daher ein Bedarf an Verbindungen, die selektiv gegen Zellzykluskinasen des Typs 1, wie CDK, sind und Zellzykluskinasen des Typs 2 inhibieren.

Weiterhin besteht ein Bedarf an Strukturen, die neben der Selektivität gegen Zellzykluskinasen des Typs 1 und Inhibierung der Zellzykluskinasen des Typs 2 das Tumorwachstum durch die Inhibierung einer oder mehrerer weiterer Kinasen hemmen (Multi-target Tumor Growth Inhibitoren = MTGI).

Bevorzugt ist die zusätzliche Inhibierung folgender Kinasefamilien:
- Rezeptortyrosinkinasen, die die Angiogenese regulieren (angiogene Rezeptortyrosinkinasen), wie beispielsweise die Rezeptortytrosinkinasen, die am Vascular Endothelial Growth Factor (VEGF) / VEGF-Rezeptor System, Fibroblast Growth Factor (FGF) / FGF-Rezeptor System, am Eph-Ligand / EphB4 System, und am Tie-Ligand / Tie System beteiligt sind,
- Rezeptortyrosinkinasen, deren Aktivität zur Proliferation von Zellen beiträgt (proliferative Rezeptortyrosinkinasen), wie beispielsweise Rezeptortyrosinkinasen, die am Platelet-Derived Growth Factor (PDGF) Ligand / PDGF-Rezeptor System, c-Kit Ligand / c-Kit-Rezeptor System und am FMS-like Tyrosinkinase 3 (Flt-3) Ligand / Flt-3 System beteiligt sind,
- Kontrollpunktkinasen (Checkpoint-Kinasen), die den geordneten Ablauf der Zellteilung überwachen, wie beispielsweise ATM und ATR, Chk1 und Chk2, Mps1, Bub1 und BubR1,
- Kinasen, deren Aktivität die Zelle vor Apoptose schützt (anti-apoptotische Kinasen, Kinasen in sog. survival pathways, anti-apoptotische Kinasen), wie beispielsweise Akt/PKB, PDK1, IkappaB kinase (IKK), Pim1, und integrin-linked kinase (ILK),
- Kinasen, die für die Migration von Tumorzellen notwendig sind (migratorische Kinasen), wie beispielsweise focal adhesion kinase (FAK), und Rho kinase (ROCK).

Den strukturell naheliegenden Stand der Technik bilden die Strukturen folgender Patentanmeldungen:

WO 2002/096888 offenbart Anilino-Pyrimidinderivate als Inhibitoren der Zyklin-abhängigen Kinasen. Ein Sulfoximinsubstituent ist für das Anilin nicht offenbart.

WO 2004/026881 offenbart makrozyklische Anilino-Pyrimidinderivate als Inhibitoren der Zyklin-abhängigen Kinasen. Ein für das Anilin möglicher Sulfoximin-Substituent ist am Stickstoffatom des Sulfoximins nur unsubstituiert offenbart.

WO 2005/037800 offenbart offene Anilino-Pyrimidinderivate als Inhibitoren der Zyklin-abhängigen Kinasen. Ein Sulfoximinsubstituent ist für das Anilin offenbart.

Allen diesen Strukturen des Standes der Technik ist gemeinsam, dass sie Zellzykluskinasen des Typs 1 inhibieren d.h. nicht selektiv gegen Zellzykluskinasen des Typs 2 sind.

Ausgehend von diesem Stand der Technik ist die Aufgabe der vorliegenden Erfindung, Inhibitoren des Zellzyklusses mit spefizisch ausgewählten Kinaseselektivitäten zur Verfügung zu stellen.

Insbesondere besteht ein Bedarf an Verbindungen, die selektiv gegen Zellzykluskinasen des Typs 1, wie Zyklin-abhängige Kinasen, sind und gleichzeitig Zellzykluskinasen des Typs 2, wie Aurora, inhibieren.

Die Aufgabe der vorliegenden Anmeldung wird gelöst durch Verbindungen der allgemeinen Formel I, die einen am Stickstoff substituierten Sulfoximinsubstituenten aufweisen.

Es wurde überraschend gefunden, dass die Substitution des Sulfoximin-Stickstoffs zu Verbindungen führt, die selektiv Zellzykluskinasen des Typs 2 inhibieren, insbesondere die Aurora inhibieren und gleichzeitig selektiv gegen Zellzykluskinasen des Typs 1, wie Zyklin-abhängige Kinasen, sind.

Die Substitution des Sulfoximinstickstoffatoms eröffnet weiterhin die Möglichkeit, Verbindungen zur Verfügung zu stellen, die neben der Inhibierung der Zellzykluskinasen des Typs 2 eine weitere Kinase inhibieren, so dass das Tumorwachstum effektiv gehemmt wird, insbesondere eine Kinase aus den Kinasefamilien der Rezeptortyrosinkinasen, der Kontrollpunktkinasen, der anti-apoptotischen Kinasen oder der migratorischen Kinasen.

Die Aufgabe der vorliegenden Erfindung wird gelöst durch Verbindungen der allgemeinen Formel I 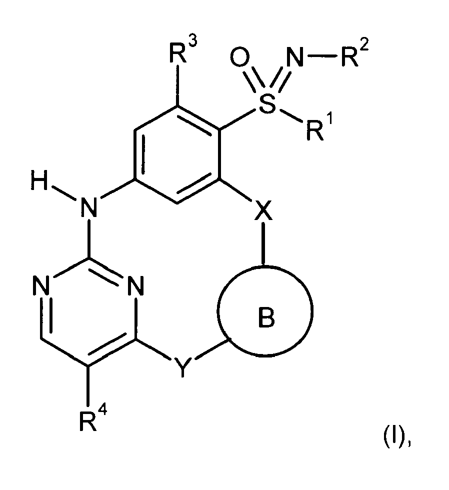 in der
- B: für eine Prop-1,3-ylen-, But-1,4-ylen-, Pent-1,5-ylen- oder Hex-1,6-ylen-gruppe steht, die ein- oder mehrfach, gleich oder verschieden substituiert sein kann mit
(i) Hydroxy, -NR¹¹R¹², Cyano, Halogen, -CF₃, -C₁-C₆-Alkoxy, -NR¹³-C(O)-C₁-C₃-Alkyl, -NR¹³-SO₂-C₁-C₃-Alkyl, -OCF₃ und/oder
(ii) einem C₁-C₆-Alkylrest, der gegebenenfalls mit Hydroxy, -NR¹¹R¹², Cyano, Halogen, -CF₃, -C₁-C₆-Alkoxy, -NR¹³-C(O)-C₁-C₃-Alkyl, -NR¹³-SO₂-C₁-C₃-Alkyl oder -OCF₃ ein- oder mehrfach, gleich oder verschieden substituiert ist,
- R¹: für
(i) einen gegebenenfalls mit Hydroxy, -NR¹¹R¹², Cyano, Halogen, C₁-C₆-Alkoxy, -CF₃ und/oder -OCF₃ ein- oder mehrfach, gleich oder verschieden substituierten C₁-C₆-Alkylrest oder
(ii) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierter C₃-C₇-Cycloalkylring oder
(iii) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierter C₆-Arylring oder
(iv) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierter Heteroarylring mit 5 oder 6 Ringatomen steht,
- R²: für R⁵, -SO₂-R⁶, -C(O)O-R⁶, -C(O)-R⁶, -C(O)-NR¹¹R¹², -C(S)-NR¹¹R¹², -Si(R⁷R⁸R⁹), -R¹⁰-Si(R⁷R⁸R⁹) oder -SO₂-R¹⁰-Si(R⁷R⁸R⁹) steht,
- R³: für
(i) Wasserstoff, Hydroxy, Halogen, Cyano, -CF₃, C₁-C₆-Alkoxy -OCF₃ oder-NR¹¹R¹² oder
(ii) einen gegebenenfalls mit Halogen, Hydroxy, C₁-C₆-Alkoxy oder der Gruppe -NR¹¹R¹² ein- oder mehrfach, gleich oder verschieden substituierten C₁-C₆-Alkylrest oder
(iii) eine gegebenenfalls mit Halogen, Hydroxy, C₁-C₆-Alkoxy oder der Gruppe -NR¹¹R¹² ein- oder mehrfach, gleich und/oder verschieden substituierte C₁-C₆-Alkoxygruppe steht oder
(iv) einen gegebenenfalls mit Halogen, Hydroxy, C₁-C₆-Alkoxy, der Gruppe -NR¹¹R¹² und/oder C₁-C₆-Alkyl ein- oder mehrfach, gleich oder verschieden substituierten C₃-C₇-Cycloalkylring steht,
- R⁴: für
(i) Halogen, Cyano, Nitro, -NR¹¹R¹², -CF₃, C₁-C₆-Alkoxy oder -OCF₃ oder
(ii) einen gegebenenfalls mit Hydroxy, -NR¹¹R¹², Cyano, Halogen, C₁-C₆-Alkoxy, -CF₃ und/oder -OCF₃ ein- oder mehrfach, gleich oder verschieden substituierten C₁-C₆-Alkyl-, C₂-C₆-Alkenyl- oder C₂-C₆-Alkinylrest oder
(iii) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierten C₆-Arylring oder
(iv) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierten Heteroarylring mit 5 oder 6 Ringatomen steht,
- X: für -S-, -S(O)-, -N H- oder -O- steht,
- Y: für -NR¹³-, -S-, -S(O)-, oder -O- steht
wobei
R⁵ ein
(i) C₁-C₆-Alkylrest oder
(ii) C₃-C₇-Cycloalkylrest oder
(iii) C₃-C₆-Alkenylrest oder
(iv) C₃-C₆-Alkinylrest oder
(v) C₆-Arylring oder
(vi) Heteroarylring mit 5 oder 6 Ringatomen sein kann, die gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituiert sein können,
R⁶ ein
(i) C₁-C₆-Alkylrest oder
(ii) C₃-C₇-Cycloalkylrest oder
(iii) C₂-C₆-Alkenylrest oder
(iv) C₂-C₆-Alkinylrest oder
(v) C₆-Arylring oder
(vi) Heteroarylring mit 5 oder 6 Ringatomen sein kann, die gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituiert sein
können,
R⁷, R⁸ und R⁹ unabhängig voneinander
(i) ein C₁-C₆-Alkylrest, und/oder
(ii) ein C₆-Arylring sein können
R¹⁰ für eine C₁-C₃-Alkylengruppe steht, und
R¹¹ und R¹² unabhängig voneinander
(i) Wasserstoff und/oder
(ii) ein C₁-C₆-Alkylrest, und/oder
(iii) ein C₆-Arylring und/oder
(iv) ein Heteroarylring mit 5 oder 6 Ringatomen sein können, wobei (ii), (iii) und (iv) gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein können,
   oder
R¹¹ und R¹² zusammen mit dem Stickstoffatom einen Heterocyclylring mit 3 bis 7 Ringatomen bilden, der gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein und ein weiteres Heteroatom enthalten kann, und
R¹³und R¹⁴ unabhängig voneinander für Wasserstoff oder einen C₁-C₆-Alkylrest stehen, der gegebenenfalls mit Hydroxy, -NH₂, Cyano, Halogen, -CF₃, C₁-C₆-Alkoxy und/oder -OCF₃ ein- oder mehrfach, gleich oder verschieden substituiert ist,
sowie deren Salze, Diastereomere und Enantiomere.

Der vorliegenden Anmeldung liegen folgende Definitionen zu Grunde:

### Cₙ-Alkylrest:

Monovalenter, geradkettiger oder verzweigter, gesättigter Kohlenwasserstoffrest mit n Kohlenstoffatomen.

### Ein C₁-C₆ Alkylrest umfasst unter anderem beipielsweise:

Methyl-, Ethyl-, Propyl-, Butyl-, Pentyl-,Hexyl-, *iso*-Propyl-, *iso*-Butyl-, *sec*-Butyl, *tert*-Butyl-, *iso*-Pentyl-, 2-Methylbutyl-, 1-Methylbutyl-, 1-Ethylpropyl-, 1,2-Dimethylpropyl, *neo*-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- 1,2-Dimethylbutyl-.

Bevorzugt ist ein Methyl-, Ethyl- oder Propylrest.

Der Alkylrest kann gegebenenfalls ein oder mehrfach, gleich oder verschieden substituiert sein mit Hydroxy, -NR¹¹R¹², Cyano, Halogen, -CF₃, C₁-C₆-Alkoxy und/oder -OCF₃.
Bevorzugt ist Hydroxy.

### cₙ-Alkenylrest:

monovalenter, geradkettiger oder verzweigter Kohlenwasserstoffrest mit n Kohlenstoffatomen und mindestens einer Doppelbindung.

### Ein C₂-C₆ Alkenylrest umfasst unter anderem beispielsweise:

Vinyl-, Allyl-, (E)-2-Methylvinyl-, (Z)-2-Methylvinyl-, Homoallyl-, (E)-But-2-enyl-, (Z)-But-2-enyl-, (E)-But-1-enyl-, (Z)-But-1-enyl-, Pent-4-enyl-, (E)-Pent-3-enyl-, (Z)-Pent-3-enyl-, (E)-Pent-2-enyl-, (Z)-Pent-2-enyl-, (E)-Pent-1-enyl-, (Z)-Pent-1-enyl-, Hex-5-enyl-, (E)-Hex-4-enyl-, (Z)-Hex-4-enyl-, (E)-Hex-3-enyl-, (Z)-Hex-3-enyl-, (E)-Hex-2-enyl-, (Z)-Hex-2-enyl-, (E)-Hex-1-enyl-, (Z)-Hex-1-enyl-, Isopropenyl-, 2-Methylprop-2-enyl-, 1-Methylprop-2-enyl-, 2-Methylprop-1-enyl-, (E)-1-Methylprop-1-enyl-, (Z)-1-Methylprop-1-enyl-, 3-Methylbut-3-enyl-, 2-Methylbut-3-enyl-, 1-Methylbut-3-enyl-, 3-Methylbut-2-enyl-, (E)-2-Methylbut-2-enyl-, (Z)-2-Methylbut-2-enyl-, (E)-1-Methylbut-2-enyl-, (Z)-1-Methylbut-2-enyl-, (E)-3-Methylbut-1-enyl-, (Z)-3-Methylbut-1-enyl-, (E)-2-Methylbut-1-enyl-, (Z)-2-Methylbut-1-enyl-, (E)-1-Methylbut-1-enyl-, (Z)-1-Methylbut-1-enyl-, 1,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, (E)-3-Methylpent-3-enyl-, (Z)-3-Methylpent-3-enyl-, (E)-2-Methylpent-3-enyl-, (Z)-2-Methylpent-3-enyl-, (E)-1-Methylpent-3-enyl-, (Z)-1-Methylpent-3-enyl-, (E)-4-Methylpent-2-enyl-, (Z)-4-Methylpent-2-enyl-, (E)-3-Methylpent-2-enyl-, (Z)-3-Methylpent-2-enyl-, (E)-2-Methylpent-2-enyl-, (Z)-2-Methylpent-2-enyl-, (E)-1-Methylpent-2-enyl-, (Z)-1-Methylpent-2-enyl-, (E)-4-Methylpent-1-enyl-, (Z)-4-Methylpent-1-enyl-, (E)-3-Methylpent-1-enyl-, (Z)-3-Methylpent-1-enyl-, (E)-2-Methylpent-1-enyl-, (Z)-2-Methylpent-1-enyl-, (E)-1-Methylpent-1-enyl-, (Z)-1-Methylpent-1-enyl-, 3-Ethylbut-3-enyl-, 2-Ethylbut-3-enyl-, 1-Ethylbut-3-enyl-, (E)-3-Ethylbut-2-enyl-, (Z)-3-Ethylbut-2-enyl-, (E)-2-Ethylbut-2-enyl-, (Z)-2-Ethylbut-2-enyl-, (E)-1-Ethylbut-2-enyl-, (Z)-1-Ethylbut-2-enyl-, (E)-3-Ethylbut-1-enyl-, (Z)-3-Ethylbut-1-enyl-, 2-Ethylbut-1-enyl-, (E)-1-Ethylbut-1-enyl-, (Z)-1-Ethylbut-1-enyl-, 2-Propylprop-2-enyl-, 1-Propylprop-2-enyl-, 2-Isopropylprop-2-enyl-, 1-Isopropylprop-2-enyl-, (E)-2-Propylprop-1-enyl-, (Z)-2-Propylprop-1-enyl-, (E)-1-Propylprop-1-enyl-, (Z)-1-Propylprop-1-enyl-, (E)-2-Isopropylprop-1-enyl-, (Z)-2-Isopropylprop-1-enyl-, (E)-1-Isopropylprop-1-enyl-, (Z)-1-Isopropylprop-1-enyl-, (E)-3,3-Dimethylprop-1-enyl-, (Z)-3,3-Dimethylprop-1-enyl-, 1-(1,1-Dimethylethyl)ethenyl.

### Bevorzugt ist ein Vinyl- oder Allylrest.

Der Alkenylrest kann gegebenenfalls ein oder mehrfach, gleich oder verschieden substituiert sein mit Hydroxy, -NR¹¹R¹², Cyano, Halogen, -CF₃, C₁-C₆-Alkoxy und/oder -OCF₃.
Bevorzugt ist Hydroxy.

### Cₙ-Alkinylrest:

Monovalenter, geradkettiger oder verzweigter Kohlenwasserstoffrest mit n Kohlenstoffatomen und mindestens einer Dreifachbindung.

### Ein C₂-C₆ Alkinylrest umfasst unter anderem beispielsweise:

Ethinyl-, Prop-1-inyl-, Prop-2-inyl-, But-1-inyl-, But-2-inyl-, But-3-inyl-, Pent-1-inyl-, Pent-2-inyl-, Pent-3-inyl-, Pent-4-inyl-, Hex-1-inyl-, Hex-2-inyl-, Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl, 2-Methylpent-4-inyl, 1-Methylpent-4-inyl, 2-Methylpent-3-inyl, 1-Methylpent-3-inyl, 4-Methylpent-2-inyl, 1-Methylpent-2-inyl, 4-Methylpent-1-inyl, 3-Methylpent-1-inyl, 2-Ethylbut-3-inyl-, 1-Ethylbut-3-inyl-, 1-Ethylbut-2-inyl-, 1-Propylprop-2-inyl-, 1-Isopropylprop-2-inyl-, 2,2-Dimethylbut-3-inyl-, 1,1-Dimethylbut-3-inyl-, 1,1-Dimethylbut-2-inyl- oder eine 3,3-Dimethylbut-1-inyl-.

Bevorzugt ist ein Ethinyl-, Prop-1-inyl- oder Prop-2-inyl-rest.

Der Alkinylrest kann gegebenenfalls ein oder mehrfach, gleich oder verschieden substituiert sein mit Hydroxy, -NR¹¹R¹², Cyano, Halogen, -CF₃, C₁-C₆-Alkoxy und/oder -OCF₃.
Bevorzugt ist Hydroxy.

### Cₙ-Alkylen:

Divalente, geradkettige oder verzweigte Kohlenwasserstoffgruppe mit n Kohlenstoffatomen.

### Eine C₁-C₆-Alkylengruppe umfasst unter anderem beispielsweise:

Methylen- (-CH₂-), Ethyliden- (-CH(CH₃)-), Ethylen- (-CH₂CH₂-), Prop-1,3-ylen-(-CH₂CH₂CH₂-), Prop-1,2-ylen- (-CH₂CH(CH₃)-), But-1,4-ylen-(-CH₂CH₂CH₂CH₂-), Pent-1,5-ylen- (-CH₂CH₂CH₂CH₂CH₂-) oder Hex-1,6-ylen-(-CH₂CH₂CH₂CH₂CH₂CH₂-).

Die Alkylengruppe kann gegebenenfalls ein oder mehrfach, gleich oder verschieden substituiert sein mit Hydroxy, -NR¹¹R¹², Cyano, Halogen, -CF₃, C₁-C₆-Alkoxy und/oder -OCF₃.
Bevorzugt ist Hydroxy.

### Für B sind folgende unverzweigte Alkylengruppen vorgesehen:

Prop-1,3-ylen- (-CH₂CH₂CH₂-), But-1,4-ylen- (-CH₂CH₂CH₂CH₂-), Pent-1,5-ylen- (-CH₂CH₂CH₂CH₂CH₂-) oder Hex-1,6-ylen- (-CH₂CH₂CH₂CH₂CH₂CH₂-).

Bevorzugt für B ist eine Prop-1,3-ylen-, But-1,4-ylen- oder eine Pent-1,5-ylen-gruppe.
Besonders bevorzugt für B ist eine But-1,4-ylen-gruppe.

Die Alkylengruppe B kann gegebenenfalls ein oder mehrfach, gleich oder verschieden substituiert sein mit Hydroxy, -NR¹¹R¹², Cyano, Halogen, -CF₃, -C₁-C₆-Alkoxy, -NR¹³-C(O)-C₁-C₃-Alkyl, -NR¹³-SO₂-C₁-C₃-Alkyl, -OCF₃ und/oder
Einem oder mehreren C₁-C₆-Alkylresten, die gegebenenfalls mit Hydroxy, -NR¹¹R¹², Cyano, Halogen, -CF₃, -C₁-C₆-Alkoxy, -NR¹³-C(O)-C₁-C₃-Alkyl, -NR¹³-SO₂-C₁-C₃-Alkyl oder -OCF₃ ein- oder mehrfach, gleich oder verschieden substituiert sind.

Bevorzugt als Substituenten für B sind Hydroxy und C₁-C₆-Alkylreste, die gegebenenfalls mit Hydroxy, -NR¹¹R¹², C₁-C₆-Alkoxy, -NR¹³-C(O)-C₁-C₃-Alkyl oder -NR¹³-SO₂-C₁-C₃-Alkyl ein- oder mehrfach, gleich oder verschieden substituiert sind.

### Cₙ-Cycloalkyl:

Monovalenter, cyclischer Kohlenwasserstoffrest mit n Kohlenstoffatomen.

### C₃-C₇-Cyclolalkylring umfasst:

Cyclopropyl, Cyclobutyl, Cyclopentyl, Cyclohexyl und Cycloheptyl.

Bevorzugt ist ein Cyclopropyl-, Cylopentyl- oder ein Cyclohexylring.

Der Cyclolalkylring kann gegebenenfalls ein oder mehrfach, gleich oder verschieden substituiert sein mit Hydroxy, -NR¹¹R¹², Cyano, Halogen, -CF₃, C₁-C₆-Alkoxy, -OCF₃ und/oder C₁-C₆-Alkyl.

### Cₙ-Alkoxy:

Geradkettiger oder verzweigter Cₙ-Alkylether der Formel -OR mit R=Alkyl.
Ein Cₙ-Alkoxyrest kann ein- oder mehrfach, gleich oder verschieden substituiert sein mit Halogen, Hydroxy, C₁-C₆-Alkoxy oder der Gruppe -NR¹¹R¹².

### Cₙ-Aryl

Cₙ-Aryl ist ein monovalentes, aromatisches Ringsystem ohne Heteroatom mit n Kohlenwasserstoffatomen.
C₆-Aryl ist gleich Phenyl.

Bevorzugt ist Phenyl.

Ein Cₙ-Arylring kann ein- oder mehrfach, gleich oder verschieden substituiert sein mit Hydroxy, -NR¹¹R¹², Cyano, Halogen, -CF₃, C₁-C₆-Alkoxy, -OCF₃ und/oder C₁-C₆-Alkyl.

### Heteroatome

Unter Heteroatomen sind Sauerstoff-, Stickstoff- oder Schwefel-Atome zu verstehen.

### Heteroaryl

Heteroaryl ist ein monovalentes, aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen aromatischen Kohlenstoffatom oder an einem Stickstoffatom sein.

### Heteroarylringe mit 5 Ringatomen umfassen beispielsweise die Ringe:

Thienyl, Thiazolyl, Furanyl, Pyrrolyl, Oxazolyl, Imidazolyl, Pyrazolyl, Isoxazolyl, Isothiazolyl, Oxadiazolyl, Triazolyl, Tetrazolyl- und Thiadiazolyl.

### Heteroarylringe mit 6 Ringatomen umfassen beispielsweise die Ringe:

Pyridyl, Pyridazinyl, Pyrimidinyl, Pyrazinyl und Triazinyl.

Ein Heteroarylring mit 5 oder 6 Ringatomen kann ein- oder mehrfach, gleich oder verschieden substituiert sein mit Hydroxy, -NR¹¹R¹², Cyano, Halogen, -CF₃, C₁-C₆-Alkoxy, -OCF₃ und/oder C₁-C₆-Alkyl.

### Heterocyclyl

Heterocyclyl im Sinne der Erfindung ist ein vollständig hydriertes Heteroaryl (vollständig hydriertes Heteroaryl = gesättigtes Heterocyclyl) d.h. ein nicht aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen Kohlenstoffatom oder an einem Stickstoffatom sein. Heterocycylring mit 3 Ringatomen umfasst beispielsweise:
Aziridinyl.
Heterocycylring mit 4 Ringatomen umfasst beispielsweise:
Azetidinyl.
Heterocycylringe mit 5 Ringatomen umfassen beispielsweise die Ringe:
Pyrrolidinyl, Imidazolidinyl und Pyrazolidinyl.
Heterocyclylringe mit 6 Ringatomen umfassen beispielsweise die Ringe:
Piperidinyl, Piperazinyl, Morpholinyl und Thiomorphinyl.
Heterocycylring mit 7 Ringatomen umfasst beispielsweise:
Azepanyl, [1,3]-Diazepanly, [1,4]-Diazepanyl.

Ein Heterocyclylring mit 3 bis 7 Ringatomen kann ein- oder mehrfach, gleich oder verschieden substituiert sein mit Hydroxy, -NR¹¹R¹², Cyano, Halogen, -CF₃, C₁-C₆-Alkoxy, -OCF₃ und/oder C₁-C₆-Alkyl.

### Halogen

Die Bezeichnung Halogen umfasst Fluor, Chlor, Brom und lod.
Bevorzugt ist Brom.

Die Substituenten -NR¹¹ R¹² und -NR¹³R¹⁴ sind gegebenenfalls substituierte Aminogruppen,
wobei
- R¹¹ und R¹²: unabhängig voneinander
(i) Wasserstoff und/oder
(ii) ein C₁-C₆-Alkylrest, und/oder
(iii) ein C₆-Arylring und/oder
(iv) ein Heteroarylring mit 5 oder 6 Ringatomen sein können, wobei (ii), (iii) und (iv) gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein können,
   oder
- R¹¹ und R¹²: zusammen mit dem Stickstoffatom einen Heterocyclylring mit 3 bis 7 Ringatomen bilden, der gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein und ein weiteres Heteroatom enthalten kann, und
- R¹³und R¹⁴: unabhängig voneinander für Wasserstoff oder einen C₁-C₆-Alkylrest stehen, der gegebenenfalls mit Hydroxy, -NH₂, Cyano, Halogen, -CF₃, C₁-C₆-Alkoxy und/oder -OCF₃ ein- oder mehrfach, gleich oder verschieden substituiert ist.

Bevorzugt sind R¹¹ und R¹² unabhängig voneinander Wasserstoff und/oder C₁-C₆-Alkylreste.

In der allgemeinen Formel I kann B sein:
eine Prop-1,3-ylen-, But-1,4-ylen-, Pent-1,5-ylen- oder Hex-1,6-ylen-gruppe,
die ein- oder mehrfach, gleich oder verschieden substituiert sein kann mit
   (i) Hydroxy, -NR¹¹R¹², Cyano, Halogen, -CF₃, C₁-C₆-Alkoxy, -NR¹³-C(O)-C₁-C₃-Alkyl, -NR¹³-SO₂-C₁-C₃-Alkyl, -OCF₃ und/oder
   (ii) einem oder mehreren C₁-C₆-Alkylresten, die gegebenenfalls mit Hydroxy, -NR¹¹R¹², Cyano, Halogen, -CF₃, C₁-C₆-Alkoxy, -NR¹³-C(O)-C₁-C₃-Alkyl, -NR¹³-SO₂-C₁-C₃-Alkyl oder -OCF₃ ein- oder mehrfach, gleich oder verschieden substituiert sind.

Als Substituenten für B bevorzugt sind Hydroxy und/oder ein oder mehreren C₁-C₆-Alkylresten, die gegebenenfalls mit Hydroxy, -NR¹¹R¹², Cyano, Halogen, -CF₃, C₁-C₆-Alkoxy, -NR¹³-C(O)-C₁-C₃-Alkyl, -NR¹³-SO₂-C₁-C₃-Alkyl oder-OCF₃ ein- oder mehrfach, gleich oder verschieden substituiert sind.

Besonders bevorzugte Substituenten für B sind Hydroxy und/oder ein oder mehreren C₁-C₆-Alkylresten, die gegebenenfalls mit Hydroxy, -NR¹¹R¹², C₁-C₆-Alkoxy, -NR¹³-C(O)-C₁-C₃-Alkyl oder -NR¹³-SO₂-C₁-C₃-Alkyl ein- oder mehrfach, gleich oder verschieden substituiert sind.

B ist bevorzugt eine Prop-1,3-ylen-, But-1,4-ylen- oder Pent-1,5-ylen-gruppe, die ein- oder mehrfach, gleich oder verschieden substituiert sein können mit Hydroxy und/oder einem oder mehreren C₁-C₆-Alkylresten, die gegebenenfalls mit Hydroxy, -NR¹¹R¹², Cyano, Halogen, -CF₃, C₁-C₆-Alkoxy, -NR¹³-C(O)-C₁-C₃-Alkyl, -NR¹³-SO₂-C₁-C₃-Alkyl oder -OCF₃ ein- oder mehrfach, gleich oder verschieden substituiert sind.

Besonders bevorzugt für B ist eine But-1,4-ylen-gruppe, die ein- oder mehrfach, gleich oder verschieden substituiert sein kann mit Hydroxy und/oder einem oder mehreren C₁-C₆-Alkylresten, die gegebenenfalls mit Hydroxy, -NR¹¹R¹², C₁-C₆-Alkoxy, -NR-(O)-C₁-C₃-Alkyl oder -NR-SO₂-C₁-C₃-Alkyl ein- oder mehrfach, gleich oder verschieden substituiert sind.
In der allgemeinen Formel I kann R¹ sein:
(i) ein gegebenenfalls mit Hydroxy, -NR¹¹R¹², Cyano, Halogen, C₁-C₆-Alkoxy, -CF₃ und/oder -OCF₃ ein- oder mehrfach, gleich oder verschieden substituierten C₁-C₆-Alkylrest oder
(ii) ein gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierter C₃-C₇-Cycloalkylring oder
(iii) ein gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierter C₆-Arylring oder
(iv) ein gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierter Heteroarylring mit 5 oder 6 Ringatomen.

R¹ ist bevorzugt ein gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituierter C₁-C₆-Alkylrest.

In der allgemeinen Formel I kann R² sein:
für R⁵, -SO₂-R⁶, -C(O)O-R⁶, -C(O)-R⁶, -C(O)-NR¹¹R¹², -C(S)-NR¹¹R¹², -Si(R⁷R⁸R⁹), -R¹⁰-Si(R⁷R⁸R⁹) oder -SO₂-R¹⁰-Si(R⁷R⁸R⁹),
   wobei
   - R⁵: ein
   (i) C₁-C₆-Alkylrest oder
   (ii) C₃-C₆-Alkenylrest oder
   (iii) C₃-C₆-Alkinylrest oder
   (iv) C₆-Arylring oder
   (v) Heteroarylring mit 5 oder 6 Ringatomen sein kann, die gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituiert sein können,
   - R⁶: ein
   (i) C₁-C₆-Alkylrest oder
   (ii) C₂-C₆-Alkenylrest oder
   (iii) C₂-C₆-Alkinylrest oder
   (iv) C₆-Arylring oder
   (v) Heteroarylring mit 5 oder 6 Ringatomen sein kann, die gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituiert sein können,
   - R⁷, R⁸ und R⁹: unabhängig voneinander
   (i) ein C₁-C₆-Alkylrest, und/oder
   (ii) ein C₆-Arylring sein können
   - R¹⁰: für eine C₁-C₃-Alkylengruppe steht,
   - R¹¹ und R¹²: unabhängig voneinander
   (i) Wasserstoff und/oder
   (ii) ein C₁-C₆-Alkylrest, und/oder
   (iii) ein C₆-Arylring und/oder
   (iv) ein Heteroarylring mit 5 oder 6 Ringatomen sein können, wobei (ii), (iii) und (iv) gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein können,
      oder
   - R¹¹ und R¹²: zusammen mit dem Stickstoffatom einen Heterocyclylring mit 3 bis 7 Ringatomen bilden, der gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein und ein weiteres Heteroatom enthalten kann, und
   - R¹³ und R¹⁴: unabhängig voneinander für Wasserstoff oder einen C₁-C₆-Alkylrest stehen, der gegebenenfalls mit Hydroxy, -NH₂, Cyano, Halogen, -CF₃, C₁-C₆-Alkoxy und/oder -OCF₃ ein- oder mehrfach, gleich oder verschieden substituiert ist.
R² ist bevorzugt R⁵, -SO₂-R⁶, -C(O)O-R⁶, -C(O)-R⁶, -C(O)-NR¹¹R¹² oder -SO₂-R¹⁰-Si(R⁷R⁸R⁹), wobei
   - R⁵, R⁶, R⁷,:
   - R⁸ und R⁹: unabhängig voneinander für einen C₁-C₆-Alkylrest steht, der gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituiert ist,
   - R¹⁰: für eine C₁-C₃-Alkylengruppe steht und
   - R¹¹ und R¹²: unabhängig voneinander
   (i) Wasserstoff und/oder
   (ii) ein C₁-C₆-Alkylrest
      sein können, wobei (ii) gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert ist, wobei
   - R¹³ und R¹⁴: unabhängig voneinander Wasserstoff oder ein C₁-C₆-Alkylrest sein können, der gegebenenfalls mit Hydroxy, -NH₂, Cyano, Halogen, -CF₃ und/oder -OCF₃ ein- oder mehrfach, gleich oder verschieden substituiert ist.

Besonders bevorzugt ist R²:
-SO₂-R⁶, -C(O)O-R⁶, -C(O)-NR¹¹R¹² oder -SO₂-R¹⁰-Si(R⁷R⁸R⁹),
wobei
R⁶, R⁷, R⁸ und R⁹ unabhängig voneinander für C₁-C₅-Alkylreste stehen,
R¹⁰ für eine C₁-C₅-Alkylengruppe steht und
R¹¹ und R¹² unabhängig voneinander Wasserstoff und/oder C₁-C₆-Alkylreste sein können.

In der allgemeinen Formel I kann R³ sein:
(i) Wasserstoff, Hydroxy, Halogen, Cyano, -CF₃, C₁-C₆-Alkoxy -OCF₃ oder-NR¹¹R¹² oder
(ii) ein gegebenenfalls mit Halogen, Hydroxy, C₁-C₆-Alkoxy oder der Gruppe -NR¹¹R¹² ein- oder mehrfach, gleich oder verschieden substituierten C₁-C₆-Alkylrest oder
(iii) eine gegebenenfalls mit Halogen, Hydroxy, C₁-C₆-Alkoxy oder der Gruppe -NR¹¹R¹² ein- oder mehrfach, gleich und/oder verschieden substituierte C₁-C₆-Alkoxygruppe steht oder
(iv) ein gegebenenfalls mit Halogen, Hydroxy, C₁-C₆-Alkoxy, der Gruppe -NR¹¹R¹² und/oder C₁-C₆-Alkyl ein- oder mehrfach, gleich oder verschieden substituierten C₃-C₇-Cycloalkylring stehen,
   wobei
   - R¹¹ und R¹²: unabhängig voneinander
   (i) Wasserstoff und/oder
   (ii) ein C₁-C₆-Alkylrest, und/oder
   (iii) ein C₆-Arylring und/oder
   (iv) ein Heteroarylring mit 5 oder 6 Ringatomen sein können,
      wobei (ii), (iii) und (iv) gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein können,
      oder
   - R¹¹ und R¹²: zusammen mit dem Stickstoffatom einen Heterocyclylring mit 3 bis 7 Ringatomen bilden, der gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein und ein weiteres Heteroatom enthalten kann, und
   - R¹³und R¹⁴: unabhängig voneinander für Wasserstoff oder einen C₁-C₆-Alkylrest stehen, der gegebenenfalls mit Hydroxy, -NH₂, Cyano, Halogen, -CF₃, C₁-C₆-Alkoxy und/oder -OCF₃ ein- oder mehrfach, gleich oder verschieden substituiert ist.

R³ ist bevorzugt:
(i) Wasserstoff, Hydroxy, Halogen, C₁-C₆Alkoxy, -NR¹¹R¹²
(ii) ein gegebenenfalls mit Halogen, Hydroxy, C₁-C₆-Alkoxy oder der Gruppe -NR¹¹R¹² ein- oder mehrfach, gleich oder verschieden substituierter -C₁-C₆-Alkylrest oder
(iii) eine gegebenenfalls mit Halogen, Hydroxy, C₁-C₆-Alkoxy oder der Gruppe -NR¹¹R¹² ein- oder mehrfach, gleich und/oder verschieden substituierte C₁-C₆-Alkoxygruppe.

R³ ist besonders bevorzugt Wasserstoff.

In der allgemeinen Formel I kann R⁴ sein:
(i) Halogen, Cyano, Nitro, -NR¹¹R¹², -CF₃, C₁-C₆-Alkoxy oder -OCF₃ oder
(ii) ein gegebenenfalls mit Hydroxy, -NR¹¹R¹², Cyano, Halogen, C₁-C₆-Alkoxy, -CF₃ und/oder -OCF₃ ein- oder mehrfach, gleich oder verschieden substituierten C₁-C₆-Alkyl-, C₂-C₆-Alkenyl- oder C₂-C₆-Alkinylrest oder
(iii) ein gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierten C₆-Arylring oder
(iv) ein gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierten Heteroarylring mit 5 oder 6 Ringatomen.
   wobei
   - R¹¹ und R¹²: unabhängig voneinander
   (i) Wasserstoff und/oder
   (ii) ein C₁-C₆-Alkylrest, und/oder
   (iii) ein C₆-Arylring und/oder
   (iv) ein Heteroarylring mit 5 oder 6 Ringatomen sein können,
      wobei (ii), (iii) und (iv) gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein können,
      oder
   - R¹¹ und R¹²: zusammen mit dem Stickstoffatom einen Heterocyclylring mit 3 bis 7 Ringatomen bilden, der gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein und ein weiteres Heteroatom enthalten kann, und
   - R¹³ und R¹⁴: unabhängig voneinander für Wasserstoff oder einen C₁-C₆-Alkylrest stehen, der gegebenenfalls mit Hydroxy, -NH₂, Cyano, Halogen, -CF₃, C₁-C₆-Alkoxy und/oder -OCF₃ ein- oder mehrfach, gleich oder verschieden substituiert ist.

R⁴ ist bevorzugt:
(i) Halogen oder -CF₃
(ii) ein gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierter C₆-Arylring oder
(iii) ein gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierter Heteroarylring mit 5 oder 6 Ringatomen steht, wobei
   - R¹¹ und R¹²: unabhängig voneinander
   (i) Wasserstoff und/oder
   (ii) ein C₁-C₆-Alkylrest
      sein können, wobei (ii) gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert ist, und
   - R¹³und R¹⁴: unabhängig voneinander für Wasserstoff oder einen C₁-C₆-Alkylrest stehen, der gegebenenfalls mit Hydroxy, -NH₂, Cyano, Halogen, -CF₃, C₁-C₆-Alkoxy und/oder -OCF₃ ein- oder mehrfach, gleich oder verschieden substituiert ist.

R⁴ ist besonders bevorzugt Halogen, insbesondere Brom oder lod

In der allgemeinen Formel I kann X sein:
- S-, -S(O)-, -NH- oder -O-.
   X ist bevorzugt -NH- oder -O-.

In der allgemeinen Formel I kann Y sein: -
-NR¹³-, -S-, -S(O)-, oder -O-,
   wobei R¹³ Wasserstoff oder ein C₁-C₆-Alkylrest sein kann, der gegebenenfalls mit Hydroxy, -NH₂, Cyano, Halogen, -CF₃, C₁-C₆-Alkoxy und/oder -OCF₃ ein- oder mehrfach, gleich oder verschieden substituiert ist.

Y ist bevorzugt -NH- oder -S-, besonders bevorzugt -NH-.

In der allgemeinen Formel 1 kann R⁵ sein:
(i) ein C₁-C₆-Alkylrest oder
(ii) ein C₃-C₆-Alkenylrest oder
(iii) ein C₃-C₆-Alkinylrest oder
(iv) ein C₆-Arylring oder
(v) ein Heteroarylring mit 5 oder 6 Ringatomen,
   die gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituiert sein können.

R⁵ ist bevorzugt ein C₁-C₆-Alkyl- oder ein C₃-C₆-Alkenylrest, die gegebenenfalls mit Hydroxy, -NR¹1R¹², Cyano, Halogen, -CF₃, C₁-C₆-Alkoxy und/oder -OCF₃ ein- oder mehrfach, gleich oder verschieden substituiert sein können.

Besonders bevorzugt ist R⁵ ein C₂-C₅-Alkylrest, der gegebenenfalls mit Hydroxy, -NR¹¹R¹² und/oder C₁-C₆-Alkoxy ein- oder mehrfach, gleich oder verschieden substituiert sein kann.

In der allgemeinen Formel I kann R⁶ sein:
(i) ein C₁-C₆-Alkylrest oder
(ii) ein C₂-C₆-Alkenylrest oder
(iii) ein C₂-C₆-Alkinylrest oder
(iv) ein C₆-Arylring oder
(v) ein Heteroarylring mit 5 oder 6 Ringatomen,
die gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituiert sein können.

R⁶ ist bevorzugt ein C₁-C₆-Alkylrest, der gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituiert sein kann.

Besonders bevorzugt ist R⁶ ein C₂-C₅-Alkylrest, der gegebenenfalls mit Hydroxy, -NR¹¹R¹², und/oder C₁-C₆-Alkoxy ein- oder mehrfach, gleich oder verschieden substituiert sein kann.

In der allgemeinen Formel I können R⁷, R⁸ und R⁹ unabhängig voneinander sein:
(i) ein C₁-C₆-Alkylrest, und/oder
(ii) ein C₆-Arylring.

Bevorzugt für R⁷, R⁸ und R⁹ sind C₁-C₆-Alkylreste.

In der allgemeinen Formel I kann R¹⁰ sein:
eine C₁-C₃-Alkylengruppe.

R¹⁰ ist bevorzugt Ethylen.

In der allgemeinen Formel I können R¹¹ und R¹² unabhängig voneinander sein:
(i) Wasserstoff und/oder
(ii) ein C₁-C₆-Alkylrest, und/oder
(iii) ein C₆-Arylring und/oder
(iv) ein Heteroarylring mit 5 oder 6 Ringatomen sein können,
wobei (ii), (iii) und (iv) gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein können,
oder
R¹¹ und R¹² bilden zusammen mit dem Stickstoffatom einen Heterocyclylring mit 3 bis 7 Ringatomen, der gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein und ein weiteres Heteroatom enthalten kann,
wobei
R¹³und R¹⁴ unabhängig voneinander für Wasserstoff oder einen C₁-C₆-Alkylrest stehen, der gegebenenfalls mit Hydroxy, -NH₂, Cyano, Halogen, -CF₃, C₁-C₆-Alkoxy und/oder -OCF₃ ein- oder mehrfach, gleich oder verschieden substituiert ist.

R¹¹ und R¹² sind bevorzugt unabhängig voneinander Wasserstoff und/oder C₁-C₆-Alkylreste.

R¹³und R¹⁴ können unabhängig voneinander sein:
Wasserstoff oder ein C₁-C₆-Alkylrest, der gegebenenfalls mit Hydroxy, -NH₂, Cyano, Halogen, -CF₃, C₁-C₆-Alkoxy und/oder -OCF₃ ein- oder mehrfach, gleich oder verschieden substituiert ist.

R¹³ und R¹⁴ sind bevorzugt unabhängig voneinander Wasserstoff und/oder ein C₁-C₆-Alkylrest.

Eine bevorzugte Untergruppe bilden Verbindungen gemäß allgemeiner Formel 1, in der
- B: für eine Prop-1,3-ylen-, But-1,4-ylen-, Pent-1,5-ylen- oder Hex-1,6-ylen-gruppe steht,
- R¹: für einen C₁-C₅-Alkylrest steht,
- R²: für -SO₂-R⁵, -C(O)O-R⁶, -C(O)-NR¹¹R¹² oder -SO₂-R¹⁰-Si(R⁷R⁸R⁹) steht, wobei
R⁵, R⁶, R⁷,
R⁸ und R⁹ unabhängig voneinander für C₁-C₅-Alkylreste stehen,
R¹⁰ für eine C₁-C₅-Alkylengruppe steht,
R¹¹ und R¹² unabhängig voneinander Wasserstoff und/oder C₁-C₆-Alkylreste sein können,
R³ für Wasserstoff steht und
R⁴ für ein Halogen steht,
sowie deren Salze, Diastereomere und Enantiomere.

Eine besonders bevorzugte Untergruppe bilden Verbindungen gemäß allgemeiner Formel I,
in der
- B: für eine But-1,4-ylen-gruppe steht,
- R¹: für eine Methylgruppe steht,
- R²: für ein -SO₂-R⁶, -C(O)O-R⁶, -C(O)-NHR⁶ oder -SO₂-C₂H₄-Si(CH₃)₃ steht, wobei
R⁶ ein Ethyl- oder Propylrest sein kann,
- R³: für Wasserstoff steht,
- R⁴: für ein Halogen steht,
- X: für -O- oder -NH- steht, und
- Y: für -NH-steht.
sowie deren Salze, Diastereomere und Enantiomere.

Ebenfalls als von der vorliegenden Erfindung als erfasst anzusehen sind alle Verbindungen, die sich ergeben durch jede mögliche Kombination der oben genannten möglichen, bevorzugten und besonders bevorzugten Bedeutungen der Substituenten.

Besondere Ausführungsformen der Erfindung bestehen darüber hinaus in Verbindungen, die sich durch Kombination der direkt in den Beispielen offenbarten Bedeutungen für die Substituenten ergeben.

Der Anmeldung liegt folgende Gruppierung von Proteinkinasen zugrunde:
A. Zellzykluskinasen des Typs 1: CDKs, Plk
B. Zellzykluskinasen des Typs 2: Aurora
C. angiogene Rezeptortyrosinkinasen: a) VEGF-R, b) Tie, c) FGF-R, d) EphB4
D. proliferative Rezeptortyrosinkinasen: a) PDGF-R, Flt-3, c-Kit
E. Kontrollpunktkinasen: a) AMT/ATR, b) Chk 1/2, c) TTK/hMps1, BubR1, Bub1
F. anti-apoptotische Kinasen a) AKT/PKB b) IKK c) PIM1, d) ILK
G. Migratorische Kinasen a) FAK, b) ROCK

### A. Zellzykluskinasen des Typs 1: CDK und Plk

Der eukaryote Zellteilungszyklus stellt die Duplikation des Genoms und seine Verteilung auf die Tochterzellen sicher, indem er eine koordinierte und regulierte Abfolge von Ereignissen durchläuft. Der Zellzyklus wird in vier aufeinanderfolgende Phasen eingeteilt: die G1 Phase repräsentiert die Zeit vor der DNA-Replikation, in der die Zelle wächst und für externe Stimuli empfänglich ist. In der S Phase repliziert die Zelle ihre DNA, und in der G2 Phase bereitet sie sich auf den Eintritt in die Mitose vor. In der Mitose (M Phase) wird die replizierte DNA getrennt und die Zellteilung vollzogen.

Die Zyklin-abhängigen Kinasen (CDKs), eine Familie von Serin/Threonin-Kinasen, deren Mitglieder die Bindung eines Zyklins (Cyc) als regulatorische Untereinheit zu ihrer Aktivierung benötigen, treiben die Zelle durch den Zellzyklus. Unterschiedliche CDK/Cyc Paare sind in den verschiedenen Phasen des Zellzyklus aktiv. Für die grundlegende Funktion des Zellzyklus bedeutende CDK/Cyc Paare sind beispielsweise CDK4(6)/CycD, CDK2/CycE, CDK2/CycA, CDK1/CycA und CDK1/CycB.

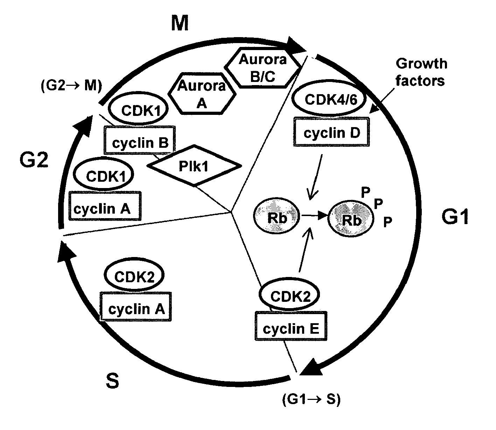

Der Eintritt in den Zellzyklus und das Durchlaufen des "Restriction Point", der die Unabhängigkeit einer Zelle von weiteren Wachstumssignalen für den Abschluss der begonnenen Zellteilung markiert, werden durch die Aktivität der CDK4(6)/CycD und CDK2/CycE Komplexe kontrolliert. Das wesentliche Substrat dieser CDK-Komplexe ist das Retinoblastoma-Protein (Rb), das Produkt des Retinoblastoma Tumorsuppressor Gens. Rb ist ein transkriptionelles Ko-Repressor Protein. Neben anderen noch weitgehend unverstandenen Mechanismen, bindet und inaktiviert Rb Transkriptionsfaktoren vom E2F-Typ, und bildet transkriptionelle Repressorkomplexe mit Histon-Deacetylasen (HDAC) (Zhang H.S. et al. (2000). Exit from G1 and S phase of the cell cycle is regulated by repressor complexes containing HDAC-Rb-hSWI/SNF and Rb-hSWI/SNF. Cell 101, 79-89). Durch die Phosphorylierung des Rb durch CDKs werden gebundene E2F Transkriptionsfaktoren freigesetzt und führen zu transkriptioneller Aktivierung von Genen, deren Produkte für die DNA Synthese und die Progression durch die S-Phase benötigt werden. Zusätzlich bewirkt die Rb-Phosphorylierung die Auflösung der Rb-HDAC Komplexe, wodurch weitere Gene aktiviert werden. Die Phosphorylierung von Rb durch CDKs ist mit dem Überschreiten des "Restriction Point" gleichzusetzen. Für die Progression durch die S-Phase und deren Abschluss ist die Aktivität der CDK2/CycE und CDK2/CycA Komplexe notwendig. Nach vollständiger Replikation der DNA steuert die CDK1 im Komplex mit CycA oder CycB das Durchlaufen der Phase G2 und den Eintritt der Zelle in die Mitose (Abb. 1). Beim Übergang von der G2 Phase in die Mitose trägt die Polo-like Kinase Plk1 zur Aktivierung der CDK1 bei. Während des Ablaufs der Mitose ist Plk1 weiterhin an der Maturierung der Zentrosomen, dem Aufbau des Spindelapparates, der Separierung der Chromosomen und der Trennung der Tochterzellen beteiligt.

### B. Zellzykluskinasen des Typs 2: Aurora Kinasen

Die Familie der Aurora Kinasen besteht im humanen Organismus aus drei Mitgliedern: Aurora-A, Aurora-B und Aurora-C. Die Aurora Kinasen regulieren wichtige Prozesse während der Zellteilung (Mitose). Aurora-A ist an den Zentrosomen und den Spindelmikrotubuli lokalisiert, wo es verschiedene Substratproteine phosphoryliert, unter anderem das Kinesin Eg5, TACC, PP1. Die genauen Mechanismen der Genese des Spindelapparates und der Rolle von Aurora-A dabei sind allerdings noch weitgehend unklar. Aurora-B ist Teil eines Multiprotein Komplexes, der an der Zentrosomenstruktur der Chromosomen lokalisiert ist und neben Aurora-B u.a. INCENP, Survivin und Borealin/Dasra B enthält (zusammenfassende Übersicht in: Vagnarelli & Earnshaw, Chromosomal passengers: the four-dimensional regulation of mitotic events. Chromosoma. 2004 Nov;113(5):211-22. Epub 2004 Sep 4). Die Kinaseaktivität von Aurora-B stellt sicher, dass vor der Teilung der Chromosomenpaare alle Verbindungen zum Mikrotubulin-Spindelapparat korrekt sind (sogenannter Spindel Checkpoint). Substrate von Aurora-B sind hier unter anderem Histon H3 und MCAK. Nach Trennung der Chromosomen ändert Aurora-B seine Lokalisation und kann während der letzten Mitosephase (Zytokinese) an der noch verbliebenen Verbindungsbrücke zwischen den beiden Tochterzellen gefunden werden. Durch Phosphorylierung seiner Substrate MgcRacGAP, Vimentin, Desmin, der leichten regulatorischen Kette von Myosin, und anderen, reguliert Aurora-B die Abschnürung der Tochterzellen. Aurora-C ist von seiner Aminosäuresequenz, Lokalisation, Substratspezifität und Funktion Aurora-B sehr ähnlich (Li X et al. Direct association with inner centromere protein (INCENP) activates the novel chromosomal passenger protein, Aurora-C. J Biol Chem. 2004 Nov 5;279(45):47201-11. Epub 2004 Aug 16;, Chen et al. Overexpression of an Aurora-C kinase-deficient mutant disrupts the Aurora-B/INCENP complex and induces polyploidy. J Biomed Sci. 2005;12(2):297-310; Yan X et al. Aurora-C is directly associated with Survivin and required for cytokinesis. Genes to ells 2005 10, 617-626). Der Hauptunterschied zwischen Aurora-B und Aurora-C ist die starke Überexpression von Aurora-C im Hoden ( Tseng TC et al. Protein kinase profile of sperm and eggs: cloning and characterization of two novel testis-specific protein kinases (AIE1, AIE2) related to yeast and fly chromosome segregation regulators. DNA Cell Biol. 1998 Oct;17(10):823-33.).
Die essentielle Funktion der Aurora Kinasen bei der Mitose macht sie zu interessanten Zielproteinen für die Entwicklung kleiner inhibitorischer Moleküle zur Behandlung von Krebs oder anderen Erkrankungen, die Störungen der Zellproliferation zur Ursache haben. Überzeugende experimentelle Daten deuten darauf hin, dass eine Inhibition der Aurora-Kinasen in vitro und in vivo das Fortschreiten zellulärer Proliferation verhindert und den programmierten Zelltod (Apoptose) auslöst. Dies konnte mittels (1) siRNA Technologie (Du & Hannon. Suppression of p160ROCK bypasses cell cycle arrest after Aurora-A/STK15 depletion. Proc Natl Acad Sci USA. 2004 Jun 15;101(24):8975-80. Epub 2004 Jun 3; Sasai K et al. Aurora-C kinase is a novel chromosomal passenger protein that can complement Aurora-B kinase function in mitotic cells. Cell Motil Cytoskeleton. 2004 Dec;59(4):249-63) oder (2) Überexpression einer dominant negativen Aurora Kinase (Honda et al. Exploring the functional interactions between Aurora B, INCENP, and survivin in mitosis. Mol Biol Cell. 2003 Aug;14(8):3325-41. Epub 2003 May 29), sowie (3) mit kleinen chemischen Molekülen gezeigt werden, die spezifisch Aurora Kinasen inhibieren (Hauf S et al. The small molecule Hesperadin reveals a role for Aurora B in correcting kinetochore-microtubule attachment and in maintaining the spindle assembly checkpoint. J Cell Biol. 2003 Apr 28;161(2):281-94. Epub 2003 Apr 21.; Ditchfield C et al. Aurora B couples chromosome alignment with anaphase by targeting BubR1, Mad2, and Cenp-E to kinetochores. J Cell Biol. 2003 Apr 28;161 (2):267-80.).
Die Inaktivierung von Aurora Kinasen führt dazu, dass (1) der mitotische Spindelapparat nicht oder fehlerhaft ausgebildet wird (vorwiegend bei Aurora-A Inhibition) und /oder dass (2) durch Blockierung des Spindel Checkpoints keine oder eine fehlerhafte Trennung der Schwesterchromatiden statt findet (vorwiegend bei Aurora-B/-C Inhibition) und / oder dass (3) die Trennung der Tochterzellen nicht vollzogen wird (vorwiegend bei Aurora-B/-C Inhibition). Diese Folgen (1-3) der Inaktivierung von Aurora Kinasen im einzelnen oder als Kombinationen führen schließlich zu Aneuploidie und / oder Polyploidie und letzlich sofort oder nach wiederholten Mitosen zu einem nicht lebensfähigen Zustand bzw. zum programmierten Zelltod der proliferierenden Zellen (mitotische Katastrophe).

Spezifische Kinaseinhibitoren können den Zellzyklus in unterschiedlichen Stadien beeinflussen. So ist beispielsweise von einem CDK4 oder einem CDK2 Inhibitor eine Blockade des Zellzyklus in der G1 Phase bzw. im Übergang von der G1 Phase zu der S-Phase zu erwarten (Zellzykluskinasen des Typs 1). Um nun die Vorteile der Inhibition der Aurora Kinasen, wie die Einleitung aberranter Mitosen die zum Zelltod führen, ausnutzen zu können, müssen Aurora Inhibitoren eine Selektivität gegenüber den Zellzykluskinasen des Typs 1 aufweisen.

### C. Angiogene Rezeptortyrosinkinasen

Rezeptortyrosinkinasen und deren Liganden sind in entscheidender Weise bei einer Vielzahl von zellulären Prozessen beteiligt, die in der Regulation des Wachstums und der Differenzierung von Zellen involviert sind. Von besonderem Interesse sind hier das Vascular Endothelial Growth Factor (VEGF) / VEGF-Rezeptor System, das Fibroblast Growth Factor (FGF) / FGF Rezeptor System, das Eph-Ligand / Eph-Rezeptor System, und das Tie-Ligand / Tie-Rezeptor System. In pathologischen Situationen, die mit einer verstärkten Neubildung von Blutgefäßen (Neovaskularisierung) einher gehen, wie z.B. Tumorerkrankungen, wurde eine erhöhte Expression von angiogenen Wachstumsfaktoren und ihrer Rezeptoren gefunden. Inhibitoren des VEGF / VEGF-Rezeptor Systems, FGF / FGF-Rezeptor Systems (Rousseau et al., The tyrp1-Tag/tyrp1-FGFR1-DN bigenic mouse: a model for selective inhibition of tumor development, angiogenesis, and invasion into the neural tissue by blockade of fibroblast growth factor receptor activity. Cancer Res. 64, :2490, 2004), des EphB4 Systems (Kertesz et al., The soluble extracellular domain of EphB4 (sEphB4) antagonizes EphB4-EphrinB2 interaction, modulates angiogenesis and inhibits tumor growth. Blood. 2005 Dec 1; [Epub ahead of print]), sowie des Tie-Ligand / Tie Systems ( Siemeister et al., Two independent mechanisms essential for tumor angiogenesis: inhibition of human melanoma xenograft growth by interfering with either the vascular endothelial growth factor receptor pathway or the Tie-2 pathway. Cancer Res. 59, 3185, 1999) können die Ausbildung eines Blutgefäßsystems in Tumoren inhibieren, damit den Tumor von der Sauerstoff- und Nährstoffversorgung abschneiden und somit das Tumorwachstum inhibieren.

### D. Proliferative Rezeptortyrosinkinasen

Rezeptortyrosinkinasen und deren Liganden sind in entscheidender Weise bei der Proliferation von Zellen beteiligt. Von besonderem Interesse sind hier das Platelet-Derived Growth Factor (PDGF) Ligand / PDGF-Rezeptor System, c-Kit Ligand / c-Kit Rezeptor System und das FMS-like Tyrosinkinase 3 (Flt-3) Ligand / Flt-3 System. In pathologischen Situationen, die mit einem verstärkten Wachstum von Zellen einher gehen, wie z.B. Tumorerkrankungen, wurde eine erhöhte Expression von proliferativen Wachstumsfaktoren und ihrer Rezeptoren oder die Kinase aktivierende Mutationen gefunden. Die Inhibition der Enzymaktivität dieser Rezeptortyrosinkinasen führt zu einer Reduktion des Tumorwachstums. Dies konnte z.B. durch Studien mit dem kleinen chemischen Molekül STI571/Glivec gezeigt werden, welches unter anderem PDGF-R und c-Kit inhibiert (zusammenfassende Übersichten in: Oestmann A., PDGF receptors - mediators of autocrine tumor growth and regulators of tumor vasculature and stroma, Cytokine Growth Factor Rev. 2004 Aug;15(4):275-86; Roskoski R., Signaling by Kit protein-tyrosine kinase - the stem cell factor receptor. Biochem Biophys Res Commun. 2005 Nov 11;337(1):1-13.; Markovic A. et al., FLT-3: a new focus in the understanding of acute leukemia. Int J Biochem Cell Biol. 2005 Jun;37(6):1168-72. Epub 2005 Jan 26.).

### E. Kontrollpunktkinasen

Unter Kontrollpunktkinasen (= Checkpoint-Kinasen) im Sinne der vorliegenden Anmeldung sind Zellzykluskinasen zu verstehen, die den geordneten Ablauf der Zellteilung überwachen, wie beispielsweise ATM und ATR, Chk1 und Chk2, Mps1, Bub1 und BubR1. Von besonderer Bedeutung sind der DNA-Schädigungs Checkpoint in der G2 Phase und der Spindel-Checkpoint während der Mitose.

Die Aktivierung der ATM, ATR, Chk1 und Chk2 Kinasen erfolgt nach DNA-Schädigung einer Zelle und führt zu einem Arrest des Zellzyklus in der G2 Phase durch Inaktivierung der CDK1. (Chen & Sanchez, Chk1 in the DNA damage response: conserved roles from yeasts to mammals. DNA Repair 3, 1025, 2004). Inaktivierung von Chk1 verursacht den Verlust des durch DNA Schädigung induzierten G2 Arrest, zur Zellzyklusprogression der Zelle in Anwesenheit geschädigter DNA, und führt schließlich zum Zelltod (Takai et al. Aberrant cell cycle checkpoint function and early embryonic death in Chk1 (-/-) mice.Genes Dev. 2000 Jun 15;14(12):1439-47; Koniaras et al. Inhibition of Chk1-dependent G2 DNA damage checkpoint radiosensitizes p53 mutant human cells. Oncogene. 2001 Nov 8;20(51):7453-63.; Liu et al. Chk1 is an essential kinase that is regulated by Atr and required for the G(2)/M DNA damage checkpoint. Genes Dev. 2000 Jun 15;14(12):1448-59.). Die Inaktivierung von Chk1, Chk2 oder Chk1 und Chk2 verhindert den durch DNA-Schädigung verursachten G2 Arrest und macht proliferierende Krebszellen empfindlicher gegenüber DNA-schädigende Therapien wie z.B. Chemotherapie oder Radiotherapie. Chemotherapien, die zur DNA Schädigung führen, sind z.B. DNA-Strangbruch induzierende Substanzen, DNA-alkylierende Substanzen, Topoisomerase Inhibitoren, Aurora Kinase Inhibitoren, Substanzen, die den Aufbau der mitotischen Spindel beeinflussen, hypoxischer Stress aufgrund limitierter Sauerstoffversorgung eines Tumors (z.B. induziert durch antiangiogene Medikamente wie VEGF Kinase Inhibitoren).

Ein zweiter wesentlicher Checkpoint innerhalb der Zellzyklus kontrolliert den korrekten Aufbau und Anhaftung des Spindelapparates an die Chromosomen während der Mitose. An diesem sogenannten Spindel-Checkpoint sind die Kinasen TTK/hMps1, Bub1, und BubR1 beteiligt (zusammenfassende Übersicht in: Kops et al. On the road to cancer: aneuploidy and the mitotic checkpoint. Nat Rev Cancer. 2005 Oct;5(10):773-85). Diese sind an Kinetochoren von noch nicht an den Spindelapparat angehefteter kondensierter Chromosomen lokalisiert und inhibieren den sogenannten anaphase-promoting complex/cyclosome (APC/C). Erst nach vollständiger und korrekter Anheftung des Spindelapparates an die Kinetochoren werden die Spindel-Checkpoint Kinasen Mps-1, Bub1, und BubR1 inaktiviert, wodurch APC/C aktiviert wird und es zur Trennung der gepaarten Chromosomen kommt. Eine Inhibition der Spindel-Checkpointkinasen führt zur der Trennung der gepaarten Chromosomen bevor alle Kinetochoren an den Spindelapparat angeheftet sind und in Folge zu chromosomalen Fehlverteilungen, die von den Zellen nicht toleriert werden und schließlich zum Zellzyklusstillstand oder zum Zelltod führen.

### F. Anti-apoptotische Kinasen

Verschiedene Mechanismen schützen eine Zelle gegenüber dem Zelltod während nicht optimaler Lebensbedingungen. In Tumorzellen führen diese Mechanismen zu einem Überlebensvorteil der Zellen in der wachsenden Tumormasse, die durch den Mangel an Sauerstoff, Glukose und weiteren Nährstoffen gekennzeichnet ist, ermöglichen ein Überleben von Tumorzellen ohne Anheftung an die extrazelluäre Matrix was zur Metastasierung führen kann, oder führen zu Resistenzen gegenüber Therapeutika. Wesentliche anti-apoptotische Signalwege umfassen den PDK1-AKT/PKB Signalweg (Altomare & Testa. Perturbations of the AKT signaling pathway in human cancer. Oncogene. 24, 7455, 2005), den NFkappaB Signalweg (Viatour et al. Phosphorylation of NFkB and IkB proteins: implications in cancer and inflammation), den Pim1 Signalweg (Hammerman et al. Pim and Akt oncogenes are independent regulators of hematopoietic cell growth and survival. Blood. 2005 105, 4477, 2005) und den integrin-linked kinase (ILK) Signalweg (Persad & Dedhar. The role of integrin-linked kinase (ILK) in cancer progression. Cancer Met. Rev. 22, 375, 2003). Durch die Inhibition der anti-apoptotischen Kinasen, wie beispielsweise AKT/PBK, PDK1, IkappaB Kinase (IKK), Pim1, oder ILK, werden die Tumorzellen gegenüber der Wirkung von Therapeutika oder gegenüber ungünstigen Lebensbedingungen in der Tumorumgebung sensitiviert. Tumorzellen werden nach Inhibition der anti-apoptotischen Kinasen empfindlicher auf durch Aurora-Inhibition verursachte Störungen der Mitose reagieren und vermehrt dem Zelltod unterliegen.

### G. Migratorische Kinasen

Invasives, Gewebe-infiltrierendes Tumorwachstum und Metastasierung setzt voraus, daß die Tumorzellen den Gewebeverband durch Migration verlassen können. Verschiedene zelluläre Mechanismen sind in die Regulation der Zellmigration involviert: Integrin-vermittelte Adhäsion an Proteine der extrazellulären Matrix reguliert über die Aktivität der focal adhesion kinase (FAK); Kontrolle des Zusammenbaus der kontraktilen Aktin-Filamente über den RhoA / Rho-Kinase (ROCK) Signalweg (zusammenfassende Übersicht in M.C. Frame, Newest findings on the oldest oncogene; how activated src does it. J. Cell Sci. 117, 989, 2004).

Die erfindungsgemäßen Verbindungen wirken zum Beispiel
- gegen Krebs, wie solide Tumore, Tumor- oder Metastasenwachstum, insbesondere:
   Ataxia-telangiectasia, Basalzellkarzinom, Blasenkarzinom, Gehirntumor, Brustkrebs, Cervix Karzinom, Tumoren des Zentralnervensystems, Kolorektalkarzinom, Endometriales Karzinom, Magenkarzinom, Gastrointestinales Karzinom, Kopf- und Halstumore, Akute lymphozytische Leukämie, Akute myelogene Leukämie, Chronische lymphozytische Leukämie, Chronische myelogene Leukämie, Haarzell Leukämie, Leberkarzinom, Lungentumor, Nicht-kleinzelliges Lungenkarzinom, Kleinzelliges Lungenkarzinom, B-Zell Lymphom, Hodgkin's Lymphom, Non-Hodgkin's Lymphom, T-Zell Lymphom, Melanom, Mesotheliom, Myelom, Myom, Tumore des Oesophagus, orale Tumore, Ovarialkarzinom, Pankreastumore, Prostatatumore, Nierenkarzinom, Sarkom, Kaposi's Sarkom, Leiomyosarkom, Hautkrebs, Plattenzellkarzinom, Hodenkrebs, Schilddrüsenkrebs, Bindegewebstumor des Magen-Darmgewebes, Bindegewebssarkom der Haut, Hypereosinophiles Syndrom, Mastzellenkrebs,
- bei kardiovaskulären Erkrankungen wie Stenosen, Arteriosklerosen und Restenosen, Stent-induzierte Restenose,
- bei Angiofibrom, Crohn-Krankheit, Endometriose, Hämangioma.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen.
Dabei ist auf Remington's Pharmaceutical Science, 15th ed. Mack Publishing Company, East Pennsylvania (1980) hinzuweisen.

Die pharmazeutischen Formulierungen können
in fester Form, zum Beispiel als Tabletten, Dragees, Pillen, Suppositorien, Kapseln, transdermale Systeme oder
in halbfester Form , zum Beispiel als Salben, Cremes, Gele, Suppositiorien, Emulsionen oder
in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein,
wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

### Herstellung der erfindungsgemäßen Verbindungen

Sulfoximine besitzen in bezug auf Struktur und Konfiguration in der Regel eine hohe Stabilität (C. Bolm, J.P. Hildebrand, J. Org. Chem. 2000, 65, 169). Diese Eigenschaften der funktionellen Gruppe erlauben oftmals auch drastische Reaktionsbedingungen und ermöglichen die einfache Derivatisierung der Sulfoximine am Imin-Stickstoff und dem α-Kohlenstoff. Enantiomerenreine Sulfoximine werden auch als Auxiliare in der diastereoselektiven Synthese verwendet ((a) S.G. Pyne, Sulfur Reports 1992, 12, 57; (b) C.R. Johnson, Aldrichchimica Acta 1985, 18, 3). Die Darstellung enantiomerenreiner Sulfoximine ist z.B. über die Racematspaltung mit enantiomerenreiner Campher-10-sulfonsäure beschrieben ((a) C.R. Johnson, C.W. Schroeck, J. Am. Chem. Soc. 1973, 95, 7418; (b) C.S. Shiner, A.H. Berks, J. Org. Chem. 1988, 53, 5543). Eine weitere Methode zur Darstellung optisch aktiver Sulfoximine besteht in der stereoselektiven Iminierung von optisch aktiven Sulfoxiden ((a) C. Bolm, P. Müller, K. Harms, Acta Chem. Scand. 1996, 50, 305; (b) Y. Tamura, J. Minamikawa, K. Sumoto, S. Fujii, M. lkeda, J. Org. Chem. 1973, 38, 1239; (c) H. Okamura, C. Bolm, Organic Letters 2004, 6, 1305).

### Verfahrensvariante 1

Die erfindungsgemäßen Verbindungen können hergestellt werden durch ein Verfahren, das durch folgende Schritte gekennzeichnet ist:
a) Funktionalisierung der 4-Position von 2,4-Dichloropyrimidinderivaten der Formel **1a** durch Umsetzung mit Nucleophilen unter basischen Bedingungen, gegebenenfalls unter Verwendung einer Schutzgruppe für die Gruppe **X,** die nach Einführung von **1b** in die 4-Position von **1a** gegebenenfalls wieder abgespalten wird, 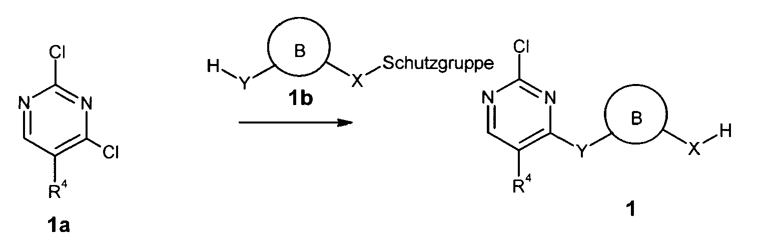
b) Oxidation einer Verbindung der Formel **2a** zum Sulfoxid der Formel **2b** 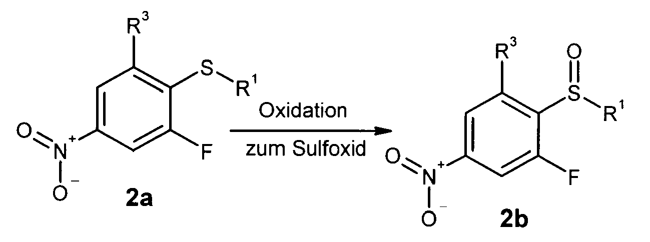
b₁) Umsetzung der Verbindung der Formel **2b** mit Natriumazid / Schwefelsäure zu einer Verbindung der Formel **2c** und N-Funktionalisierung des Sulfoximins zu einer Verbindung der Formel **2** 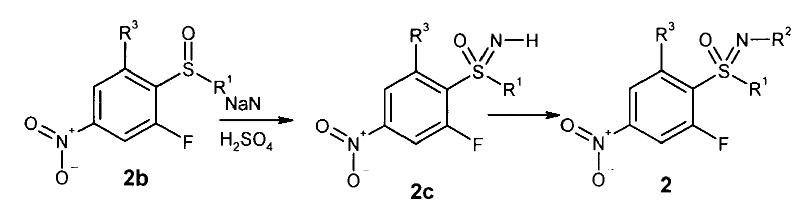 oder
b₂) direkte Umsetzung des Sulfoxides der Formel **2b** zu einer Verbindung der Formel **2**, 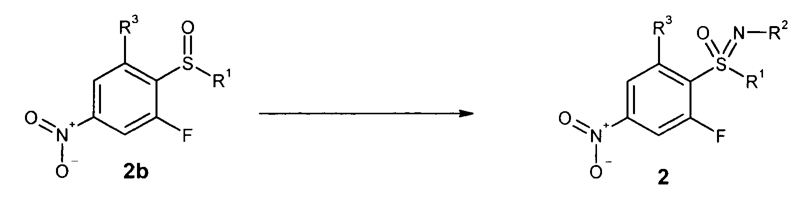
c) Umsetzung der Verbindung der Formel **1** aus Verfahrensschritt a) mit der Verbindung der Formel **2** aus Verfahrensschritt b) über eine nukleophile aromatischen Substitution zu einer Verbindung der Formel **3** 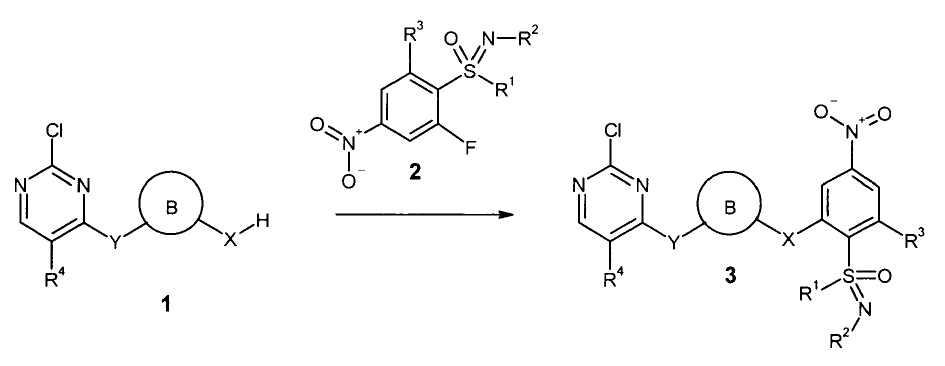
d) Reduktion der Verbindung der Formel **3** zu einer Verbindung der Formel **4** 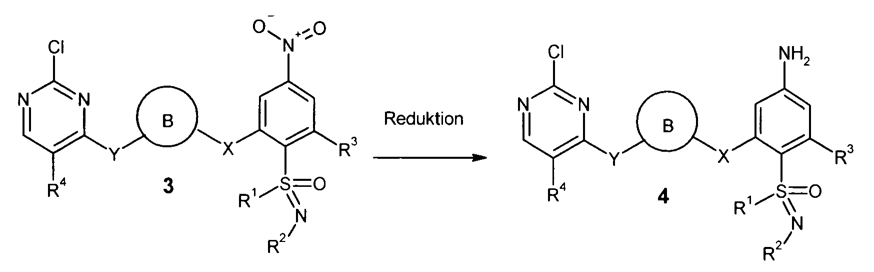
e) Cyclisierung der Verbindung der Formel **4** unter sauren Bedingungen zu einer Verbindung der Formel I

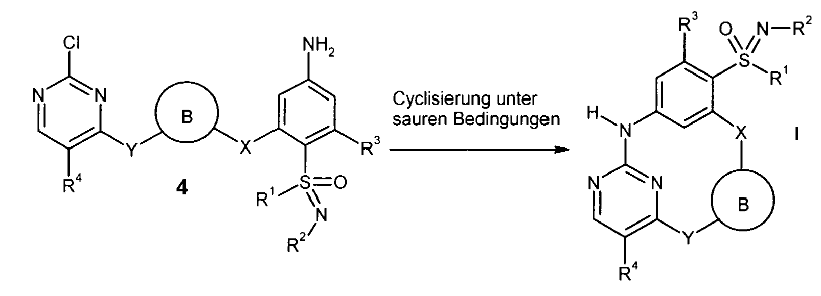

### Verfahrensschritt a)

2,4-Dichloropyrimidinderivate der Formel **1a** können durch die Umsetzung mit Nucleophilen unter basischen Bedingungen in der 4-Position funktionalisiert werden (siehe z.B.: a) U. Lücking, M. Krüger, R. Jautelat, G. Siemeister, WO 2005037800; b) U. Lücking, M. Krueger, R. Jautelat, O. Prien, G. Siemeister, A. Ernst, WO 2003076437; c) T. Brumby, R. Jautelat, O. Prien, M. Schäfer, G. Siemeister, U. Lücking, C. Huwe, WO 2002096888).
Für N-Nucleophile (Y = NH) ist besonders Acetonitril als Lösungsmittel und Triethylamin als Base geeignet. Die Umsetzung erfolgt bevorzugt bei Raumtemperatur.
Für O-Nucleophile (Y = O) ist besonders THF als Lösungsmittel und Natriumhydrid als Base geeignet. Die Umsetzung erfolgt bevorzugt bei 0°C bis Raumtemperatur.
Für S-Nucleophile (Y = S) ist besonders Acetonitril als Lösungsmittel und Triethylamin als Base geeignet. Die Umsetzung erfolgt bevorzugt bei -20°C bis Raumtemperatur.
In Abhängigkeit von der Natur der Substituenten **X** und **Y** ist ggf. die Verwendung einer geeigneten Schutzgruppe (PG) für die Gruppe **X** erforderlich. Die Schutzgruppe (PG) wird nach erfolgreicher Einführung von **1b** in die 4-Position von **1a** gegebenenfalls. wieder abgespalten (siehe z.B. T.W. Greene, P.G.M. Wuts, Protective Groups in Organic Synthesis, 2nd Edition, John Wiley & Sons, 1991).

### Verfahrensschritte b und b₁)

Eine Verbindung der Formel **2a** wird zunächst zum Sulfoxid der Formel **2b** oxidiert. Für die Überführung eines Thioethers in ein Sulfoxid stehen zahlreiche Methoden zur Verfügung (siehe z.B.: a) M.H. Ali, W.C. Stevens, Synthesis 1997, 764-768; b) I. Fernandez, N. Khiar, Chem. Rev. 2003, 103, 3651-3705). Besonders geeignet für die Darstellung von Verbindungen der Formel **2b** ist die beschriebene Verwendung von Perjodsäure / Eisen(III)chlorid. Eine Verbindung der Formel **2b** kann z.B. unter Verwendung von Natriumazid / Schwefelsäure (siehe auch: M. Reggelin, C. Zur, Synthesis 2000, 1, 1) zu einer Verbindung der Formel **2c** umgesetzt werden.
Für die weitere N-Funktionalisierung des Sulfoximins **2c** unter Bildung von Verbindungen der Formel **2** stehen diverse Methoden zur Verfügung:
- Alkylierung (siehe z.B.: C.R. Johnson, J. Org. Chem. 1993, 58, 1922-1923).
- Acylierung (siehe z.B.: a) C.P.R. Hackenberger, G. Raabe, C. Bolm, Chem. Europ. J. 2004, 10, 2942-2952; b) C. Bolm, C.P.R. Hackenberger, O. Simic, M. Verrucci, D. Müller, F. Bienewald, Synthesis 2002, 7, 879-887; c) C. Bolm, G. Moll, J.D. Kahmann, Chem. Europ. J. 2001, 7, 1118-1128).
- Arylierung (siehe z.B.:a) C. Bolm, J.P. Hildebrand, Tetrahedron Lett.1998, 39, 5731-5734; b) C. Bolm, J.P. Hildebrand, J. Org. Chem. 2000, 65, 169-175; c) C. Bolm, J.P. Hildebrand, J. Rudolph, Synthesis 2000, 7, 911-913; d) Y.C. Gae, H. Okamura, C. Bolm, J. Org. Chem. 2005, 70, 2346-2349).
- Umsetzung mit Isocyanaten / Isothiocyanaten (siehe z.B.: a) V.J. Bauer, W.J. Fanshawe, S.R. Safir, J. Org. Chem. 1966, 31, 3440-3441; b) C.R. Johnson, M. Haake, C.W. Schroeck, J. Am. Chem. Soc. 1970, 92, 6594-6598; c) S. Allenmark, L. Nielsen, W.H. Pirkle, Acta Chem. Scand. Ser. B 1983, 325-328)
- Umsetzung mit Sulfonylchloriden (siehe z.B.: a) D.J. Cram, J. Day, D.R. Rayner, D.M von Schriltz, D.J. Duchamp, D.C. Garwood. J. Am. Chem. Soc. 1970, 92, 7369-7384), b) C.R. Johnson, H.G. Corkins, J. Org. Chem. 1978, 43, 4136-4140; c) D. Craig, N.J. Geach, C.J. Pearson, A.M.Z. Slawin, A.J.P. White, D.J. Williams, Tetrahedron 1995, 51, 6071-6098).
- Umsetzung mit Chloroformiaten oder Anhydriden (siehe z.B.: a) D.J. Cram, J. Day, D.R. Rayner, D.M von Schriltz, D.J. Duchamp, D.C. Garwood. J. Am. Chem. Soc. 1970, 92, 7369-7384), b) S.G. Pyne, Z. Dong, B.W. Skelton, A.H. Allan, J. Chem. Soc. Chem. Commun. 1994, 6, 751-752; c) C.R. Johnson, H.G. Corkins, J. Org. Chem. 1978, 43, 4136-4140; d) Y.C. Gae, H. Okamura, C. Bolm, J. Org. Chem. 2005, 2346-2349).
- Silylierung: (siehe z.B.: A.J. Pearson, S.L. Blystone, H. Nar, A.A. Pinkerton, B.A. Roden, J. Yoon, J. Am. Chem. Soc. 1989, 111, 134-144).

### Verfahrensschritt b₂)

Eine weitere Möglichkeit N-funktionalisierte Verbindungen der Formel **2** zu synthetisieren, ist die direkte Umsetzung eines Sulfoxides der Formel **2b,** beispielsweise unter Verwendung folgender Reagenzien / Methoden:
- TsN₃((a) R. Tanaka, K. Yamabe, J. Chem. Soc. Chem. Commun. 1983, 329; (b) H. Kwart, A.A. Kahn, J. Am. Chem. Soc. 1967, 89, 1959))
- N-tosylimino phenyl iodinan und kat. Mengen Cu(I)triflat (J.F.K. Müller, P. Vogt, Tetrahedron Lett. 1998, 39, 4805)
- Boc-Azid und kat. Mengen Eisen(II)chlorid (T. Bach, C. Korber, Tetrahedron Lett. 1998, 39, 5015) oder
- o-Mesitylensulfonylhydroxylamin (MSH) (C.R. Johnson, R.A. Kirchhoff, H.G. Corkins, J. Org. Chem. 1974, 39, 2458)
- [N-(2-(Trimethylsilyl)ethanesulfonyl)imino]phenyliodinane (Phl=NSes) (S. Cren, T.C. Kinahan, C.L. Skinner and H. Tye, Tetrahedron Lett. 2002, 43, 2749).
- Trifluoracetamid oder Sulfonylamiden in Kombination mit lodobenzol-diacetat, Magnesiumoxid und katalytischen Mengen von Rhodium(II)-acetat dimer (H. Okamura, C. Bolm, Organic Letters 2004, 6, 1305.
- Sulfonylamiden in Kombination mit lodobenzol-diacetat und katalytischen Mengen eines chelatisierenden Ligandens und Silbersalzen (G.Y. Cho, C. Bolm, Organic Letters 2005, 7, 4983).
- NsNH₂ und lodobenzol-diacetat (G.Y. Cho, C. Bolm, Tetrahedron Lett. 2005, 46, 8007).

### Verfahrensschritt c)

Nach Verfahrensvariante 1 werden zunächst die Verbindungen der Formel **1** und der Formel **2** über eine nukleophile aromatischen Substitution (siehe z.B.: a) F.A. Carey, R.J. Sundberg, Organische Chemie, VCH, Weinheim, 1995, 1341-1359; b) Organikum, VEB Deutscher Verlag der Wissenschaften, Berlin, 1976, 421-430) zu einer Verbindung der Formel 3 umgesetzt. Besonders geeignet sind dabei polar aprotische Lösungsmittel wie z.B. DMF oder DMSO. Die zu verwendenden Basen können in Abhängigkeit von der Natur des Nukleophils variiert werden: für X = NH ist z.B. Triethylamin geeignet, für X = O ist z.B. NaH geeignet und für X = S können z.B. NaH, Triethylamin oder Kaliumcarbonat verwendet werden.

### Verfahrensschritt d)

Für die anschließende Reduktion der aromatischen Nitrogruppe zu einer Verbindung der Formel **4** stehen prinzipiell eine Reihe von Reaktionsbedingungen zur Verfügung (siehe z.B.: R.C. Larock, Comprehensive Organic Transformations, VCH, New York, 1989, 411-415). Besonders geeignet ist die beschriebene Verwendung von Titan(III)chlorid.

### Verfahrensschritt e)

Die Verbindung der Formel **4** wird abschließend in der Gegenwart einer Säure, wie z.B. Hydrogenchlorid, zu einer Verbindung der Formel I cyclisiert. In Abhängigkeit von der Natur der Verbindung der Formel **4** können verschiedene Lösungsmittel / Lösungsmittelgemische verwendet werden. Besonders geeignet ist z.B. die Verwendung von Acetonitril oder Acetonitril / Wasser. Die Reaktionstemperatur kann in Abhängigkeit von der Reaktivität der Verbindung der Formel **4,** sowie der verwendeten Säue und des verwendeten Lösungsmittels im Bereich von Raumtemperatur bis Reflux variiert werden. Für Acetonitril und Acetonitril / Wasser Gemische in Kombination mit Hydrogenschlorid als Säure ist der Temperaturbereich von 60-90°C besonders geeignet.

### Verfahrensvariante 2

Die erfindungsgemäßen Verbindungen, in denen X für -O- steht, können hergestellt werden durch ein Verfahren, das durch folgende Schritte gekennzeichnet ist:
a) Umsetzung eines Alkohole der Formel **6** mit einem Phenol der Formel 7 unter Mitsunobu Bedingungen 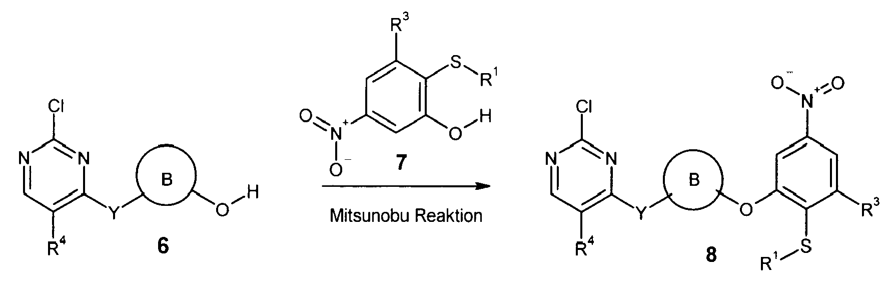
b)
   (i) Oxidation des Thioethers der Formel **8** zum Sulfoxid und anschließend
   (ii) Umsetzung zum Sulfoximin der Formel **9**. 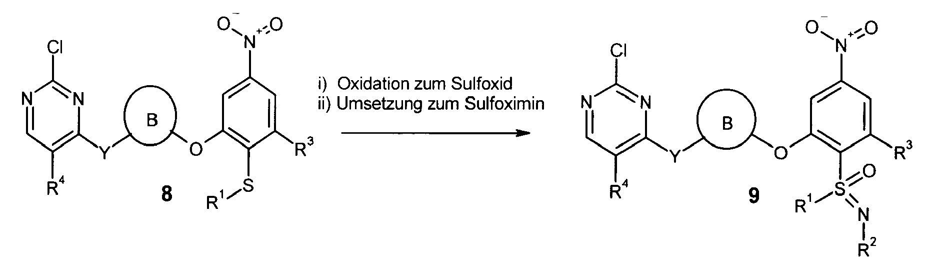
c)
   (i) Reduktion der Verbindung der Formel **9** und
   (ii) Cyclisierung unter sauren Bedingungen zu Verbindungen der Formel II.

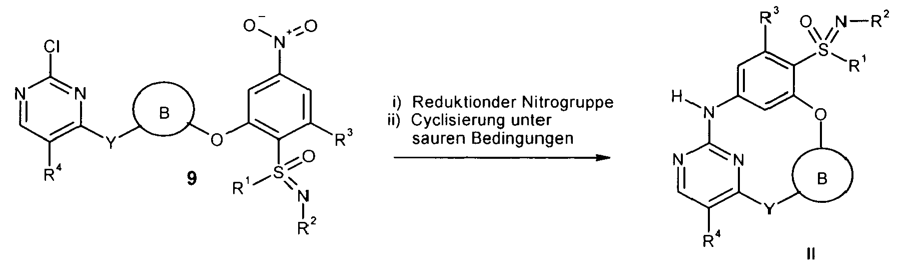

### Verfahrensschritt a)

Nach Verfahrensvariante 2 werden Alkohole des Formel **6** mit Phenolen der Formel **7** unter Mitsunobu Bedingungen (siehe z.B.: a) O. Mitsunobu, M. Yamada, T. Mukaiyama, Bull. Chem. Soc. Jpn. 1967, 40, 935; b) O. Mitsunobu, Synthesis 1981, 1; c) D.L. Hughes, 'The Mitsunobu Reaction', Organic Reactions, John Wiley & Sons, Ltd, 1992, 42, 335) zu Verbindungen der Formel **8** gekuppelt.

### Verfahrensschritt b)

(i) Zunächst erfolgt die Oxidation des Thioethers der Formel **8** zum Sulfoxid. Für die Überführung eines Thioethers in ein Sulfoxid stehen zahlreiche Methoden zur Verfügung (siehe z.B.: a) M.H. Ali, W.C. Stevens, Synthesis 1997, 764-768; b) I. Fernandez, N. Khiar, Chem. Rev. 2003, 103, 3651-3705). Besonders geeignet ist z.B. die beschriebene Verwendung von Perjodsäure / Eisen(III)chlorid.
(ii) Im Anschluss erfolgt die Umsetzung zum Sulfoximin der Formel **9**. Besonders bevorzugte Methoden sind hier beispielweise die Umsetzung des Sulfoxides mit [N-(2-(Trimethylsilyl)ethanesulfonyl)imino]phenyliodinane (Phl=NSes) (S. Cren, T.C. Kinahan, C.L. Skinner and H. Tye, Tetrahedron Lett. 2002, 43, 2749) oder Trifluoracetamid oder Sulfonylamiden in Kombination mit lodobenzol-diacetat, Magnesiumoxid und katalytischen Mengen von Rhodium(II)-acetat dimer (H. Okamura, C. Bolm, Organic Letters 2004, 6, 1305).

### Verfahrensschritt c)

(i) Für die anschließende Reduktion der aromatischen Nitrogruppe stehen prinzipiell eine Reihe von Reaktionsbedingungen zur Verfügung (siehe z.B.: R.C. Larock, Comprehensive Organic Transformations, VCH, New York, 1989, 411-415). Besonders geeignet ist die beschriebene Verwendung von Titan(III)chlorid.
(ii)Abschließend erfolgt die Cyclisierung unter sauren Bedingungen zu Verbindungen der Formel **II.**

### Verfahrensvariante 3:

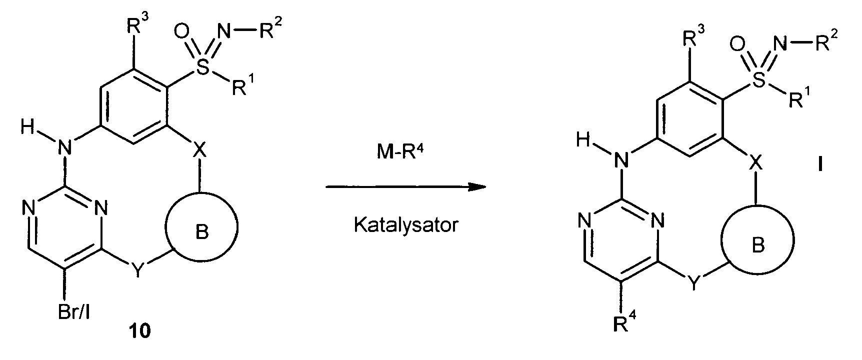

Nach Verfahrensvariante 3 werden 5-Brom oder 5-lod Derivate der Formel **10**
- im Sinne einer Suzuki Kupplung (siehe z.B.: a) F. Bellina, A. Carpita, R. Rossi. Synthesis 2004, 15, 2419; b) V. Wittmann, Nachrichten aus der Chemie 2002, 50, 1122; c) A. Herrmann, Applied Homogeneous Catalysis with Organometallic Compounds (2nd Edition) 2002, 1, 591; d) A. Suzuki in F. Diederich, P.J. Stang (Eds.) Metal-catalyzed Cross-Coupling Reactions, Wiley-VCH, New York, 1998, 47) mit Boronsäurederivaten (M = B(OH)₂ oder B(OR)₂) oder
- im Sinne einer Stille Kupplung (siehe z.B.: a) Oliver Reiser, Chemie in unserer Zeit 2001, 35, 94; b) V. Farina, V. Krishnamurthy, W.J. Scott, Org. React. (N.Y.) 1997, 50, 1) mit Zinnderivaten (M = SnR₃) oder
- im Sinne einer Negishi-Kupplung (sieh z.B.: a) E. Negishi, X. Zeng,; Metal-Catalyzed Cross-Coupling Reactions (2nd Edition) 2004, 2, 815; b) E. Negishi Handbook of Organopalladium Chemistry for Organic Synthesis 2002, 1, 229-) mit Zinkderivaten (M = ZnR) umgesetzt.

Die nachstehenden Beispiele dienen der näheren Erläuterung der Erfindung, ohne die Erfindung auf diese zu beschränken.

### Verfahrensvariante 1

### Beispiel 1 (RS)-S-[1⁵-Brom-2,4,9-triaza-1(2,4)-pyrimidina-3(1,3)-benzenacyclononaphan-3⁴-yl]-N-(ethoxycarbonyl)-S-methylsulfoximid

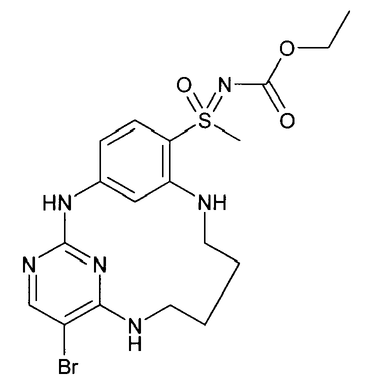

### 1. Herstellung der Zwischenprodukte

### a) Verbindung 1.1 :

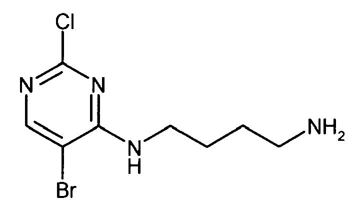

Eine Suspension von 5,37 g (23,6 mmol) 5-Brom-2,4-dichlor-pyrimidin und 4,88 g (25,9 mmol) N-BOC-1,4-diamino-butan in 102 ml Acetonitril wird unter Wasserkühlung mit 6.84 ml (49,4 mmol) Triethylamin versetzt. Das Reaktionsgemisch wird über Nacht bei Raumtemperatur gerührt und anschließend in NaCl-Lösung gegeben. Man extrahiert mit Essigester (3x). Die vereinten organischen Phasen werden getrocknet (Na₂SO₄), filtriert und eingeengt. Der erhaltene Rückstand wird mit 250 ml Acetonitril und 45 ml einer 4 N Lösung von Chlorwasserstoff in Dioxan versetzt und 2 Stunden bei Raumtemperatur gerührt. Der Ansatz wird zur Trockene einrotiert. Man versetzt mit Toluol und engt erneut zur Trockene ein. Man erhält 8,50 g des Rohproduktes als Hydrochlorid, das ohne weitere Reinigung eingesetzt wird.

### b) Verbindung 1.2

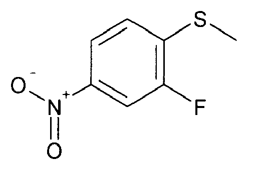

Eine eisgekühlte Lösung von 25,7 g (161,5 mmol) 1,2-Difluor-4-nitro-benzol in 172 ml DMF wird portionsweise mit 13,8 g (196,6 mmol) Natriumthiomethylat versetzt und anschließend 24 Stunden bei Raumtemperatur gerührt. Der Ansatz wird auf Eiswasser gegeben und mit Essigester extrahiert (3x). Die vereinten organischen Phasen werden getrocknet (Na₂SO₄), filtriert und eingeengt. Das erhaltene Rohprodukt wird ohne weitere Reinigung eingesetzt.

¹H-NMR (DMSO): 8.02 (m, 2H), 7.48 (m, 1 H), 2.57 (s, 3H).

### c) Verbindung 1.3

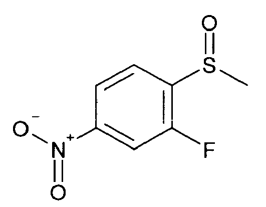

Ein Ansatz mit 25,9 g (138,5 mmol) Verbindung 1.2 und 644 mg (4,0 mmol) Eisen(III)chlorid in 112 ml Acetonitril wird bei Raumtemperatur mit 33,8 g (148 mmol) Periodsäure versetzt. Durch Wasserkühlung wird die Reaktionstemperatur unter 30°C gehalten. Die Suspension wird 1 Stunde bei RT gerührt und anschließend in ein Gemisch aus 250 ml DCM, 750 ml Eiswasser und 150 g Natriumthiosulfat Pentahydrat gegeben. Der Ansatz wird mit DCM extrahiert (3x). Die vereinten organischen Phasen werden getrocknet (Na₂SO₄), filtriert und eingeengt. Das erhaltene Rohprodukt wird aus Essigester / Hexan umkristallisiert. Man erhält 15,7 g (77,2 mmol; entsprechend 56 % der Theorie) des Produktes.

¹H-NMR (DMSO): 8.35 (m, 2H), 8.00 (m, 1 H), 2.91 (s, 3H).

### d) Verbindung 1.4

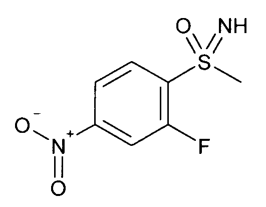

Eine eisgekühlte Suspension von 15,7 g (77,2 mmol) Verbindung 1.3 und 10,0 g (154,3 mmol) Natriumazid in 27 ml Chloroform wird unter Rühren tropfenweise mit 20,6 ml konz. Schwefelsäure versetzt. Der Ansatz wird langsam auf 45°C erwärmt und anschließend 24 Stunden bei dieser Temperatur gerührt. Nach dem Erkalten wird der Ansatz auf 800 ml Eiswasser gegeben und mit festem NaOH basisch gestellt. Es wird mit festem NaCl abgesättigt und mit DCM extrahiert (3x). Die vereinten organischen Phasen werden mit gesättigter NaCl Lösung gewaschen (2x), getrocknet (Na₂SO₄), filtriert und eingeengt. Man erhält 15,3 g des Rohproduktes, das ohne weitere Reinigung eingesetzt wird.

¹H-NMR (DMSO): 8.35 (m, 1 H), 8.24 (m, 1 H), 8.08 (m, 1 H), 5.07 (s, 1 H), 3.22 (s, 3H).

### e) Verbindung 1.5

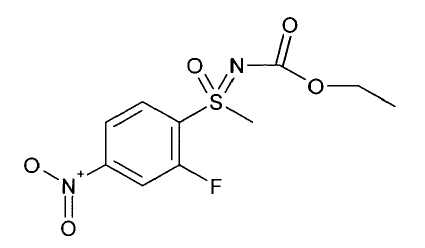

Eine eisgekühlte Lösung von 5,0 g (22,9 mmol) Verbindung 1.4 in 215 ml Pyridin wird unter Rühren tropfenweise mit 10,2 ml (106,4 mmol) Chlorameisensäureethylester versetzt. Der Ansatz wird über Nacht auf Raumtemperatur erwärmt und anschließend in gesättigte NaCI-Lösung gegeben. Es wird mit Essigester extrahiert (3x). Die vereinten organischen Phasen werden getrocknet (Na₂SO₄), filtriert und eingeengt. Man erhält 6,54 g des Rohproduktes, das ohne weitere Reinigung eingesetzt wird.

¹H-NMR (DMSO): 8.43 (m, 1 H), 8.29 (m, 1 H), 8.15 (m, 1 H), 3.87 (m, 2H), 3.55 (s, 3H), 1.05 (tr, 3H).

### f) Verbindung 1.6

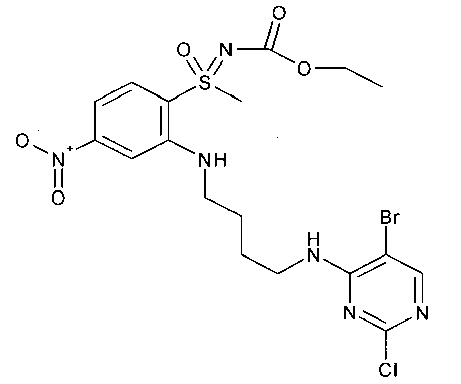

Eine Suspension von 1,50 g (5,2 mmol) Verbindung 1.5 und 2,45 g (7,8 mmol) Verbindung 1.1 in 15 ml Acetonitril wird unter Argon mit 2,2 ml (15,6 mmol) Triethylamin versetzt und 5 min bei Raumtemperatur gerührt. Anschließend wird der Ansatz auf 60°C erwärmt und 8 Stunden bei dieser Temperatur gerührt. Nach dem Abkühlen wird der Ansatz in gesättigte NaCI-Lösung gegeben und 3 x mit Essigester extrahiert. Die vereinten organischen Phasen werden getrocknet (Na₂SO₄), filtriert und eingeengt. Der verbleibende Rückstand wird chromatographisch gereinigt (Hexan / Essigester 1:1). Man erhält 2,03 g (3,7 mmol; entsprechend 71 % der Theorie) des Produktes.

¹H-NMR (DMSO): 8.19 (s, 1H), 7.86 (m, 1H), 7.73 (tr, 1H), 7.46 (m, 2H), 6.54 (tr, 1 H), 3.86 (m, 2H), 3.44 (s, 3H), 3.35 (m, 4H), 1.60 (m, 4H), 1.04 (tr, 3H).

### g) Verbindung 1.7 :

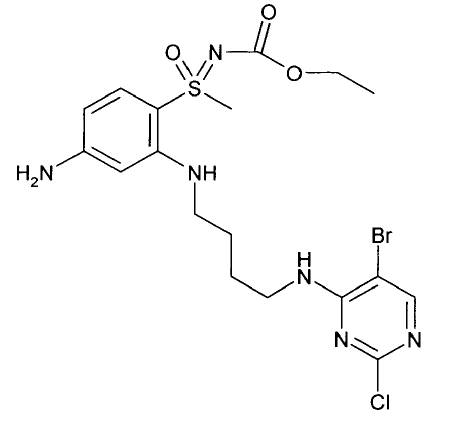

Eine Lösung von 1,0 g (1,82 mmol) Verbindung 1.6 in 40 ml THF wird unter Argon bei 0°C über einen Zeitraum von 20 Minuten mit 19 ml einer ca. 10%igen Lösung von Titan(III)Chlorid, in 20-30%iger Salzsäure versetzt. Der Ansatz wird langsam auf Raumtemperatur erwärmt. Nach 4 Stunden wird der Ansatz im Eisbad abgekühlt und mit 1 N NaOH-Lösung auf pH 7-8 gestellt. Man extrahiert gegen Essigester (3x). Die vereinten organischen Phasen werden getrocknet (Na₂SO₄), filtriert und eingeengt. Der verbleibende Rückstand wird chromatographisch gereinigt (DCM / EtOH 9:1). Man erhält 736 mg (1,42 mmol, entsprechend 78 % der Theorie) des Produktes.

¹H-NMR (DMSO): 8.19 (s, 1 H), 7.73 (tr, 1 H), 7.21 (m, 1 H), 6.07 (tr, 1 H), 5.88 (m, 4H), 3.88 (q, 2H), 3.38 (m, 2H), 3.20 (s, 3H), 3.03 (m, 2H), 1.60 (m, 4H), 1.06 (tr, 3H).

### 2. Herstellung des Endproduktes

Eine Lösung von 557 mg (1,07 mmol) Verbindung 1.7 in Acetonitril / Wasser / Methanol (35 ml / 3,5 ml / 3,5 ml) wird mittels Spritzenpumpe innerhalb von 3 Stunden zu einer Lösung von Acetonitril / Wasser / 4 N Lösung von Chlorwasserstoff in Dioxan (156 ml / 17 ml / 1,7 ml) bei 60°C gegeben. Nach 68 Stunden wird der Ansatz einrotiert und der erhaltene Rückstand chromatographisch gereinigt (DCM / EtOH 9:1). Man erhält 223 mg (0,46 mmol, entsprechend 43 % der Theorie) des Produktes.

¹H-NMR (DMSO): 9.80 (s, 1 H), 8.61 (br, 1 H), 8.08 (s, 1 H), 7.74 (br, 1 H), 7.37 (m, 1 H), 6.47 (m, 1 H), 6.38 (br, 1 H), 3.88 (q, 2H), 3.38 (m, 4H), 3.27 (s, 3H), 1.78 (m, 2H), 1.63 (m, 2H), 1.06 (tr, 3H).

MS: 483 (ES+).

### Beispiel 2

Das Racemat aus Beispiel 1 wird mittels chiraler HPLC präperativ in die Enantiomere getrennt:

| | |
|---|---|
| Säule: | Chiralpak AD-H 5µ; 250 x 20 mm |
| Eluenten: | Hexan / Ethanol; isokratisch 50% Ethanol |
| Fluss: | 10,0 ml/min |
| Detektor: | UV 300 nM |
| Temperatur: | RT |
| Retention: | Enantiomer 1 : 24,94 min |

### Beispiel 3

Das Racemat aus Beispiel 1 wird mittels chiraler HPLC präperativ in die Enantiomere getrennt:

| | |
|---|---|
| Säule: | Chiralpak AD-H 5µ; 250 x 20 mm |
| Eluenten: | Hexan / Ethanol; isokratisch 50% Ethanol |
| Fluss: | 10,0 ml/min |
| Detektor: | UV 300 nM |
| Temperatur: | RT |
| Retention: | Enantiomer 2 : 38,69 min |

### Beispiel 4 (RS)-N-(Ethoxycarbonyl)-S-[1⁵-iod-2,4,9-triaza-1(2,4)-pyrimidina-3(1,3)-benzenacyclononaphan-3⁴-yl]-S-methylsulfoximid:

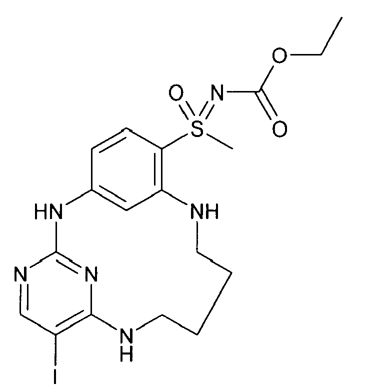

### 1. Herstellung der Zwischenprodukte:

### a) Verbindung 4.1 :

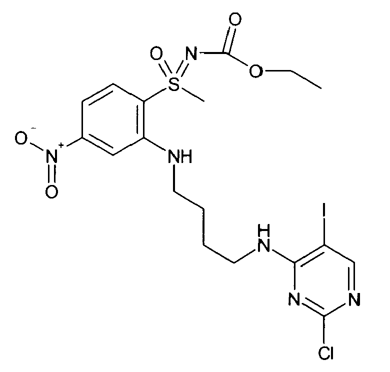

Gemäß der Vorschrift zur Darstellung von Verbindung 1.6 erhält man bei der Umsetzung von 1,20 g (4,10 mmol) der Verbindung 1.5 mit 1,75 g (4,8 mmol) N-(2-Chlor-5-lod-pyrimidin-4-yl)-propan-1,3-diamin Hydrochlorid das Produkt in 98%iger Ausbeute (2,40 g; 4,02 mmol)).

¹H-NMR (DMSO): 8.28 (s, 1 H), 7.88 (m, 1 H), 7.48 (m, 2H), 7.35 (tr, 1 H), 6.52 (tr, 1 H), 3.88 (m, 2H), 3.42 (s, 3H), 3.30 (m, 4H), 1.59 (m, 4H), 1.03 (tr, 3H).

### b) Verbindung 4.2 :

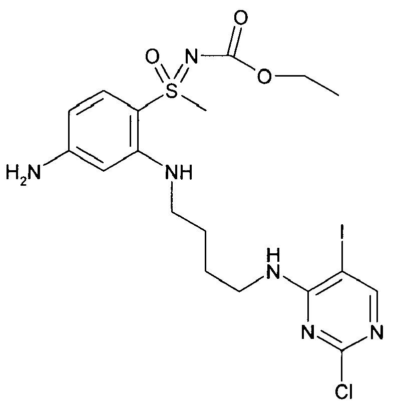

Gemäß der Vorschrift zur Darstellung von Verbindung 1.7 erhält man bei der Umsetzung von 1,20 g (2,01 mmol) Verbindung 4.1 das Produkt in 62%iger Ausbeute (0,70 g; 1,24 mmol).

¹H-NMR (DMSO): 8.28 (s, 1 H), 7.34 (tr, 1 H), 7.20 (m, 1 H), 6.05 (tr, 1 H), 5.88 (m, 4H), 3.87 (q, 2H), 3.35 (m, 2H), 3.19 (s, 3H), 3.03 (m, 2H), 1.53 (m, 4H), 1.06 (tr, 3H).

MS: 567 (ES+).

### 2. Herstellung des Endproduktes

Eine Lösung von 350 mg (0,61 mmol) Verbindung 4.2 in 10 ml Acetonitril wird mittels Spritzenpumpe innerhalb von 3 Stunden zu einer Lösung von Acetonitril / Wasser / 4 N Lösung von Chlorwasserstoff in Dioxan (45,0 ml / 5,0 ml / 0,5 ml) bei 60°C gegeben. Nach 16 Stunden wird der Ansatz einrotiert und der erhaltene Rückstand chromatographisch gereinigt (DCM / EtOH 9:1). Man erhält 160 mg (0,30 mmol, entsprechend 49 % der Theorie) des Produktes.

¹H-NMR (DMSO): 9.53 (s, 1 H), 8.69 (br, 1 H), 8.12 (s, 1 H), 7.33 (m, 1 H), 7.08 (tr, 1 H), 6.47 (m, 1 H), 6.35 (tr, 1 H), 3.88 (m, 2H), 3.30 (m, 7H), 1.59 (m, 4H), 1.08 (tr, 3H).

MS: 531 (ES+).

### Beispiel 5 (RS)-S-[1⁵-Brom-2,4,9-triaza-1(2,4)-pyrimidina-3(1,3)-benzenacyclononaphan-3⁴-yl]-S-methyl-N-[2-(trimethylsilyl)ethylsulfonyl]sulfoximid:

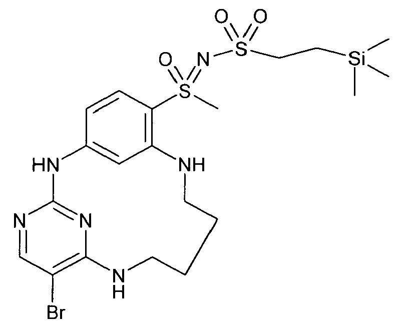

### 1. Herstellung der Zwischenprodukte

### a) Verbindung 5.1 (374011, UL 1768):

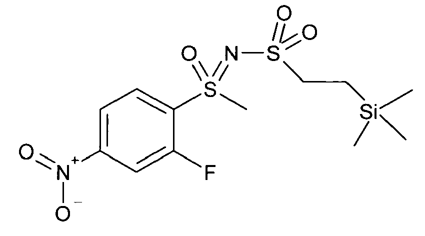

Eine Lösung von 1,90 g (8,7 mmol) Verbindung 1.4, 1,5 ml (10,5 mmol) Triethylamin und 106 mg (0,87 mmol) DMAP in 25 ml DCM wird unter Wasserkühlung über einen Zeitraum von 10 Minuten mit einer Lösung von 2,12 g (10,5 mmol) SES-CI (L.L. Parker, N.D. Gowans, S.W. Jones, D.J. Robin; Tetrahedron 2003, 59, 10165) in 25 ml DCM versetzt. Der Ansatz wird 3 Stunden bei Raumtemperatur gerührt und anschließend mit verdünnter NaCl versetzt. Man extrahiert mit Essigester (3x). Die vereinten organischen Phasen werden über einen Whatman Filter filtriert und eingeengt. Der erhaltene Rückstand wird chromatographisch (Hexan / Essigester 1:1) gereinigt. Man erhält 1,70 g (4,5 mmol; entsprechend 51 % der Theorie) des Produktes.

¹H-NMR (DMSO): 8.51 (m, 1 H), 8.30 (m, 1 H), 8.16 (m, 1 H), 3.71 (s, 3H), 2.95 (m, 2H), 0.91 (m, 2H), 0.01 (s, 9H).

### b) Verbindung 5.2 :

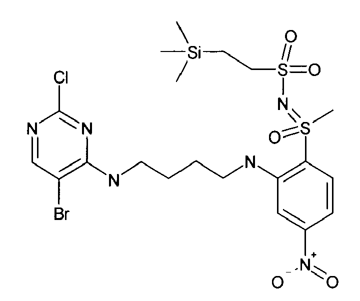

Gemäß der Vorschrift zur Darstellung von Verbindung 1.6 erhält man bei der Umsetzung von 1,50 g (4,10 mmol) der Verbindung 5.1 mit 1,86 g (5,88 mmol) Verbindung 1.1 das Produkt in 48%iger Ausbeute (1,21 g; 4,02 mmol)).

¹H-NMR (DMSO): 8.19 (s, 1 H), 7.88 (m, 1 H), 7.68 (tr, 1 H), 7.49 (m, 2H), 6.49 (tr, 1 H), 3.58 (s, 3H), 3.35 (m, 4H), 2.99 (m, 2H), 1.64 (m, 4H), 0.93 (m, 2H), -0,01 (s, 9H).

MS: 641 (ES+).

### c) Verbindung 5.3 :

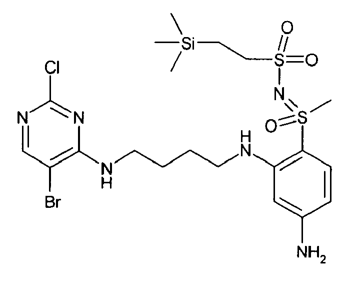

Gemäß der Vorschrift zur Darstellung von Verbindung 1.7 erhält man bei der Umsetzung von 1,21 g (1,88 mmol) Verbindung 5.2 das Produkt in 11%iger Ausbeute (0,13 g; 0,20 mmol).

¹H-NMR (DMSO): 8.20 (s, 1 H), 7.72 (tr, 1 H), 7.28 (m, 1 H), 5.98 (m, 5H), 3.37 (m, 5H), 3.07 (m, 2H), 2.96 (m, 2H), 1.64 (m, 4H), 0.92 (m, 2H), -0.02 (s, 9H).

MS: 611 (ES+).

### 2. Herstellung des Endproduktes

Eine Lösung von 65 mg (0,11 mmol) Verbindung 5.3 in 3 ml DCM wird mittels Spritzenpumpe innerhalb von 3 Stunden zu einer Lösung von Acetonitril / Wasser / 4 N Lösung von Chlorwasserstoff in Dioxan (45,0 ml / 5,0 ml / 0,5 ml) bei 70°C gegeben. Nach 24 Stunden wird der Ansatz einrotiert und der erhaltene Rückstand chromatographisch gereinigt (DCM / EtOH 9:1). Man erhält 59 mg (0,10 mmol, entsprechend 96 % der Theorie) des Produktes.

¹H-NMR (DMSO): 9.70 (s, 1 H), 8.72 (br, 1 H), 8.05 (s, 1 H), 7.54 (br, 1 H), 7.39 (m, 1 H), 6.50 (m, 1 H), 6.30 (br, 1 H), 3.30 (m, 7H), 2.93 (m, 2H), 1.65 (m, 4H), 0.92 (m, 2H), -0.02 (s, 9H).

MS: 575 (ES+).

### Beispiel 6 (RS)-S-[1⁵-Brom-2,4,9-triaza-1 (2,4)-pyrimidina-3(1,3)-benzenacyclononaphan-3⁴-yl]-N-(ethylcarbamoyl)-S-methylsulfoximid:

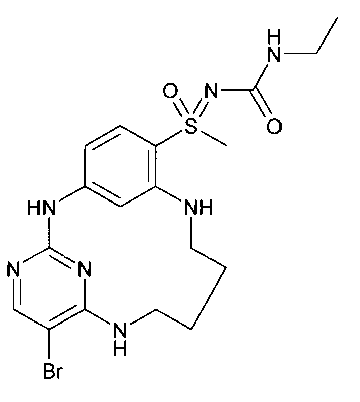

### 1. Herstellung der Zwischenprodukte

### a) Verbindung 6.1 :

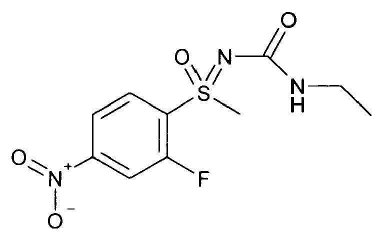

Eine Lösung von 1,32 g (6,0 mmol) Verbindung 1.4, 0,48 ml Ethylisocyanat und 0,8 ml (6,0 mmol) Triethylamin in 80 ml DCM wird 5 Tage bei 40°C gerührt. Man versetzt mit weiteren 0,25 ml (3,0 mmol) Ethylisocyanat und 0,4 ml (6,0 mmol) Triethylamin. Nach 3 Tagen wird der Ansatz eingeengt und der Rückstand chromatographisch (DCM / EtOH 95:5) gereinigt. Man erhält 1,30 g (4,49 mmol; entsprechend 75% der Theorie) des Produktes.

¹H-NMR (DMSO): 8.35 (m, 1 H), 8.24 (m, 1 H), 8.11 (m, 1 H), 7.08 (tr, 1 H), 3.41 (s, 3H), 2.85 (m, 2H), 0.89 (tr, 3H).

MS: 290 (ES+).

### b) Verbindung 6.2 :

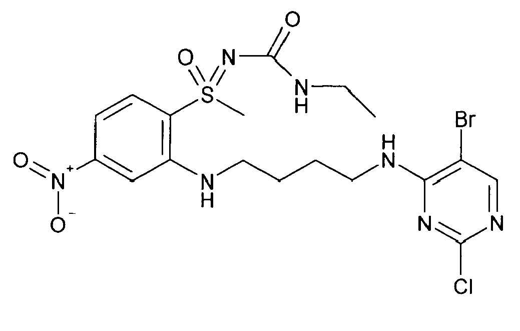

Gemäß der Vorschrift zur Darstellung von Verbindung 1.6 erhält man bei der Umsetzung von 289 mg (1,50 mmol) Verbindung 6.1 mit 419 mg (1,5 mmol) Verbindung 1.1, sowie wässriger Aufarbeitung mit verdünnter Zitronensäure das Rohprodukt in quantitativer Ausbeute (593 mg).

¹H-NMR (DMSO): 8.17 (s, 1 H), 7.83 (m, 1 H), 7.72 (tr, 1 H), 7.48 (m, 2H), 7.10 (tr, 1 H), 6.62 (tr, 1 H), 3.35 (m, 4H), 3.30 (s, 3H), 2.91 (m, 2H), 1.61 (m, 4H), 0.92 (tr, 3H).

MS: 548 (ES+).

### c) Verbindung 6.3 :

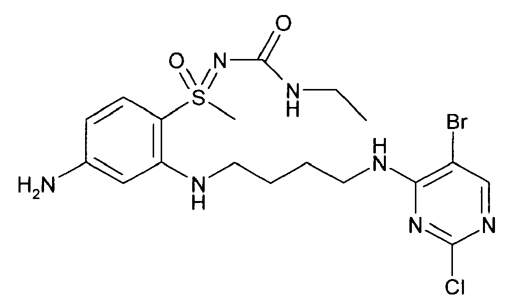

Gemäß der Vorschrift zur Darstellung von Verbindung 1.7 erhält man bei der Umsetzung von 590 mg (1,07 mmol) Verbindung 6.2 das Produkt in 39%iger Ausbeute (216 mg, 0,42 mmol).

¹H-NMR (DMSO): 8.19 (s, 1 H), 7.73 (tr, 1 H), 7.22 (m, 1 H), 6.85 (tr, 1 H), 6.21 (tr, 1H), 5.95 (m, 1 H), 5.84 (m, 3H), 3.38 (m, 2H), 3.17 (s, 3H), 2.98 (m, 4H), 1.60 (m, 4H), 0.93 (tr, 3H).

MS: 518 (ES+).

### 2. Herstellung des Endproduktes

Eine Lösung von 117 mg (0,23 mmol) Verbindung 6.3 in 5 ml Acetonitril wird mittels Spritzenpumpe innerhalb von 3 Stunden zu einer Lösung von Acetonitril / Wasser / 4 N Lösung von Chlorwasserstoff in Dioxan (45,0 ml / 5,0 ml / 0,5 ml) bei 80°C gegeben und weitere 19 Stunden gerührt. Nach dem Abkühlen wird der Ansatz mit NaHCO₃ Lösung versetzt und mit Essigester extrahiert (3x). Die vereinten organischen Phasen werden über einem Whatman Filter filtriert und eingeengt. Der erhaltene Rückstand wird chromatographisch (DCM / EtOH 95:5) gereinigt. Man erhält 44 mg (0,09 mmol; entsprechend 40% der Theorie) des Produktes.

¹H-NMR (DMSO): 9.55 (s, 1 H), 8.70 (br, 1 H), 8.03 (s, 1 H), 7.39 (tr, 1 H), 7.31 (m, 1 H), 6.96 (tr, 1 H), 6.59 (br, 1 H), 6.44 (m, 1 H), 3.35 (m, 4H), 3.19 (s, 3H), 2.92 (m, 2H), 1.65 (m, 4H), 0.93 (tr, 3H).

MS: 482 (ES+)

### Beispiel 7 (RS)-S-[1⁵-Brom-2,4,9-triaza-1(2,4)-pyrimidina-3(1,3)-benzenacyclononaphan-3⁴-yl]-S-methyl-N-(propylsulfonyl)sulfoximid:

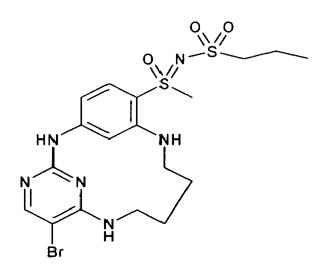

### 1. Herstellung der Zwischenprodukte

### a) Verbindung 7.1

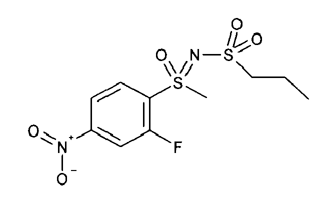

Unter Rühren wird eine Lösung von 1000 mg (4,58 mmol) Verbindung 1.4 in 30 ml Pyridin unter Argon mit 0,56 ml (5,04 mmol) Propan-1-sulfonylchlorid versetzt. Der Ansatz wird 5 Stunden bei Raumtemperatur gerührt. Man versetzt mit 0,7 ml (5,04 mmol) Triethylamin und rührt über Nacht. Der Ansatz wird erneut mit 0,52 ml (4,68 mmol) Propan-1-sulfonylchlorid versetzt und eine weitere Nacht bei Raumtemperatur gerührt. Man versetzt mit verdünnter Zitronensäure und extrahiert mit Essigester (3x). Die vereinten organischen Phasen werden mit NaHCO₃-Lösung und NaCl-Lösung gewaschen, getrocknet (Na₂SO₄), filtriert und eingeengt. Der erhaltene Rückstand wird chromatographisch (Hexan / Essigester 1:1) gereinigt. Man erhält 500 mg (1,56 mmol; entsprechend 34% der Theorie) des Produktes.

MS: 325 (ES+).

### b) Verbindung 7.2

Herstellung analog der Vorschriften zu Verbindungen 1.6 und 1.7.

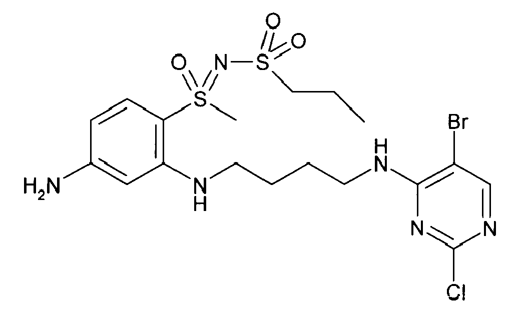

¹H-NMR (DMSO): 8.19 (s, 1 H), 7.72 (tr, 1 H), 7.25 (m, 1 H), 5.98 (m, 3H), 5.88 (m, 2H), 3.35 (m, 5H), 3.03 (m, 4H), 1.61 (m, 6H), 0.92 (tr, 3H).

MS: 553 (ES+).

### 2. Herstellung des Endproduktes

Eine Lösung von 99 mg (0,18 mmol) Verbindung 7.2 in 10 ml Acetonitril wird mittels Spritzenpumpe innerhalb von 3 Stunden zu einer Lösung von Acetonitril / Wasser / 4 N Lösung von Chlorwasserstoff in Dioxan (45,0 ml / 5,0 ml / 0,5 ml) bei 70°C gegeben und weitere 40 Stunden gerührt. Nach dem Abkühlen wird der Ansatz mit NaHCO₃-Lösung versetzt und mit Essigester extrahiert (3x). Die vereinten organischen Phasen werden getrocknet (Na₂SO₄), filtriert und eingeengt. Der erhaltene Rückstand wird chromatographisch (DCM / EtOH 95:5) gereinigt. Man erhält 41 mg (0,08 mmol; entsprechend 44% der Theorie) des Produktes.

¹H-NMR (DMSO): 9.67 (s, 1 H), 8.80 (br, 1 H), 8.09 (s, 1 H), 7.45 (m, 2H), 6.54 (m, 1 H), 6.33 (tr, 1 H), 3.45 (m, 7H), 3.07 (m, 2H), 1.72 (m, 6H), 0.98 (tr, 3H).

MS: 517 (ES+).

### Beispiel 8 (RS)-S-[1⁵-Brom-4-oxa-2,9-diaza-1(2,4)-pyrimidina-3(1,3)-benzenacyclononaphan-3⁴-yl]-S-methyl-N-(propoxycarbonyl)sulfoximid:

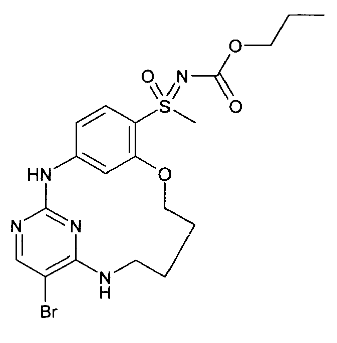

### 1. Herstellung der Zwischenprodukte

### a) Verbindung 8.1 :

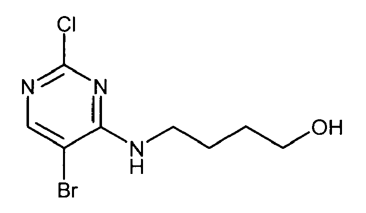

Eine Lösung von 2,28 g (10,0 mmol) 5-Brom-2,4-dichlor-pyrimidin und 1,7 ml (12,0 mmol) Triethylamin in 10 ml Acetonitril wird bei 0°C mit 1,1 ml (12,0 mmol) 4-Amino-butanol versetzt. Das Reaktionsgemisch wird durch Entfernen des Eisbades langsam unter Rühren auf Raumtemperatur erwärmt. Nach 16 Stunden wird der gebildete Niederschlag abfiltriert. Das Filtrat wird vollständig eingeengt und mit Diisopropylether digeriert. Man erhält 2,74 g (9,8 mmol, entsprechend 98% der Theorie) des Produktes.

¹H-NMR (DMSO): 8.19 (s, 1 H), 7.72 (t, 1 H), 4.45 (br, 1 H), 3.38 (m, 4H), 1.56 (m, 2H), 1.45 (m, 2H).

MS: 279 (EI).

### b) Verbindung 8.2 :

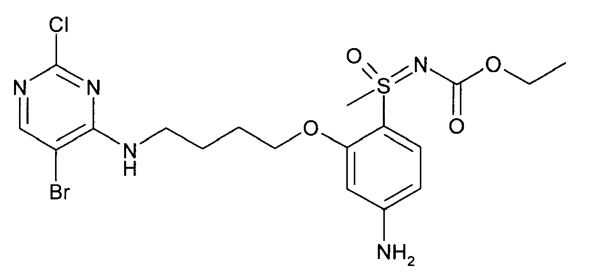

1,51 g (5,35 mmol) Verbindung 8.1 und 252 mg Natriumhydrid (55-65%) werden unter Argon eingewogen und anschließend mit 15 ml DMF versetzt. Der Ansatz wird 10 min bei Raumtemperatur gerührt und anschließend mit einer Lösung von 1,76 g (6,1 mmol) Verbindung 1.5 in 20 ml DMF versetzt. Man rührt über Nacht und gibt den Ansatz in eine gesättigte NaCl-Lösung. Man extrahiert mit Essigester (3x). Die vereinten organischen Phasen werden mit gesättigter NaCl-Lösung gewaschen, getrocknet (Na₂SO₄), filtriert und zur Trockene eingeengt. Der erhaltene Rückstand wird chromatographisch (DCM / EtOH 96:4) gereinigt. Man erhält 1,20 g des Produktes, das durch eine weitere Komponente verunreinigt ist. Das Produktgemisch wird unter Argon in 40 ml THF gelöst, und man versetzt unter Rühren mit 8,5 ml einer ca. 10%igen Lösung von Titan(III)Chlorid in 20-30%iger Salzsäure. Der Ansatz wird 3 Stunden bei Raumtemperatur gerührt. Über einen Zeitraum von 90 Minuten versetzt man portionsweise mit weiteren 3 ml der ca. 10%igen Lösung von Titan(III)Chlorid in 20-30%iger Salzsäure. Der Ansatz wird mit Essigester verdünnt und anschließend mit NaHCO₃-Lösung basisch gestellt. Man extrahiert mit Essigester (3x). Die vereinten organischen Phasen werden getrocknet (Na₂SO₄), filtriert und eingeengt. Der erhaltene Rückstand wird chromatographisch (DCM / EtOH 9:1) gereinigt. Man erhält 0,30 g (0,58 mmol; entsprechend 11 % der Theorie) des Produktes.

¹H-NMR (DMSO): 8.20 (s, 1 H), 7.73 (tr, 1 H), 7.38 (m, 1 H), 6.22 (m, 2H), 6.11 (s, 2H), 3.98 (m, 2H), 3.79 (m, 2H), 3.35 (m, 2H), 3.24 (s, 3H), 1.68 (m, 4H), 1.00 (tr, 3H).

MS: 520 (ES+).

### 2. Herstellung des Endproduktes

158 mg (0,30 mmol) Verbindung 8.2 in 200 ml Propanol werden unter Rühren bei 70 °C über einen Zeitraum von 5 Stunden mittels einer Spritzenpumpe mit einer Lösung von 9 ml Propanol / 1 ml 4 N Lösung von Chlorwasserstoff in Dioxan versetzt. Der Ansatz wird anschließend 40 Stunden bei 70°C gerührt und dann zur Trockene eingeengt. Der erhaltene Rückstand wird per HPLC gereinigt. Man erhält 73 mg (0,15 mmol, entsprechend 48 % der Theorie) des Produktes.

¹H-NMR (DMSO): 9.91 (s, 1 H), 9.08 (m, 1 H), 8.07 (s, 1 H), 7.57 (m, 2H), 6.81 (m, 1 H), 4.41 (m, 2H), 3.72 (tr, 2H), 3.39 (m, 2H), 3.31 (s, 3H), 1.75 (m, 4H), 1.42 (m, 2H), 0.75 (tr, 3H).

### HPLC:

| | |
|---|---|
| Säule: | Purospher Star C18 5µ; 125 x 25 mm |
| Eluenten: | A: H₂O + 0,1 % TFA, B:MeCN; |
| Gradient: | 76%A+24%B(1')->24->38%B(10')->95%B(0,1') |
| Fluss: | 25 ml/min |
| Detektor: | UV 254 nm; MS-ESI+ |
| Temperatur: | RT |
| Retention: | 8,8-9,6 min (Peak: 497 m/z). |

### Verfahrensvariante 2

### Beispiel 9 (RS)-S-[1⁵-Brom-4-oxa-2,9-diaza-1(2,4)-pyrimidina-3(1,3)-benzenacyclononaphan-3⁴-yl]-S-methyl-N-[2-(trimethylsilyl)ethylsulfonyl]sulfoximid

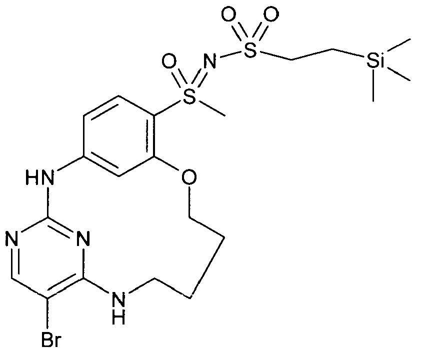

### 1. Herstellung der Zwischenprodukte

### a) Verbindung 9.1

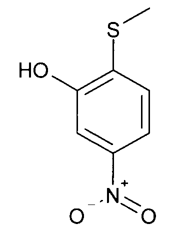

Eine Suspension von 1,0 g (14,3 mmol) Natriumthiomethylat in 3 ml N-Methylpyrrolidon (NMP) wird bei 35-40°C mit 1,67 g (10,0 mmol) 3,4-Methylendioxynitrobenzol versetzt. Der Ansatz wird nach 30 Minuten auf Eiswasser gegeben und mit Essigsäure neutralisiert. Der gebildete Niederschlag wird abgesaugt, mit Wasser gewaschen und getrocknet. Man erhält 1,7 g (9,1 mmol; entsprechend 91% der Theorie) des Produktes.

¹H-NMR (DMSO): 10.98 (s, 1 H), 7.72 (m, 1 H), 7.57 (m, 1 H), 7.29 (m, 1 H), 2.48 (s, 3H).

### b) Verbindung 9.2

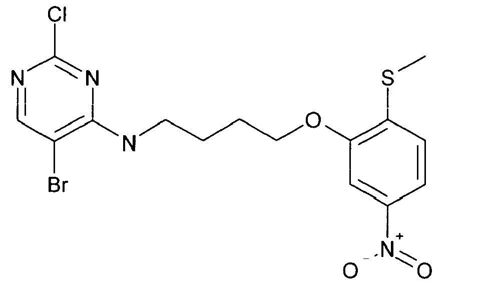

Ein Ansatz von 606 mg (2,02 mmol) Verbindung 8.1, 332 mg (1,82 mmol) Verbindung 9.1 und 641 mg (2,44 mmol) Triphenylphosphin in 25 ml THF wird bei 0°C unter Argon mit 0,42 ml DEAD Reagenz versetzt. Der Ansatz wird über Nacht gerührt, über Kieselgel eingeengt und chromatographisch (Hexan / Essigester 4:1) gereinigt. Man erhält 568 mg (1,27 mmol; entsprechend 63% der Theorie) des Produktes.

¹H-NMR (DMSO): 8.20 (s, 1 H), 7.83 (m, 2H), 7.65 (m, 1 H), 7.32 (m, 1 H), 4.21 (m, 2H), 3.43 (m, 2H), 2,50 (s, 3H), 1.75 (m, 4H).

### c) Verbindung 9.3

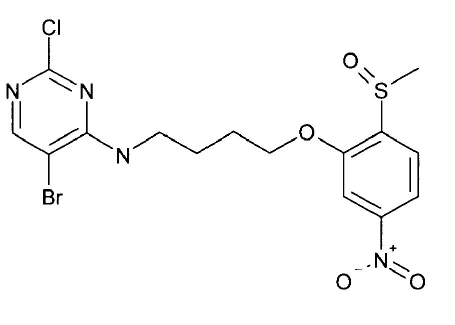

Ein Ansatz von 470 mg (1,05 mmol) Verbindung 9.2 in 30 ml Acetonitril wird bei Raumtemperatur mit 170 mg (1,05 mmol) Eisen(III)chlorid und 264 (1,15 mmol) Periodsäure versetzt. Der Ansatz wird 20 Minuten bei Raumtemperatur gerührt und anschießend in NaHCO₃-Lösung gegeben. Man extrahiert mit Essigester (3x). Die vereinten organischen Phasen werden mit NaCl-Lösung gewaschen, getrocknet (Na₂SO₄), filtriert und eingeengt. Man erhält 468 mg des Rohproduktes, das ohne weitere Reinigung eingesetzt wird.

¹H-NMR (DMSO): 8.22 (s, 1 H), 8.07 (m, 1 H), 7.82 (m, 3H), 4.30 (m, 2H), 3.43 (m, 2H), 2.79 (s, 3H), 1.73 (m, 4H).

MS: 463 (ES+).

### d) Verbindung 9.4

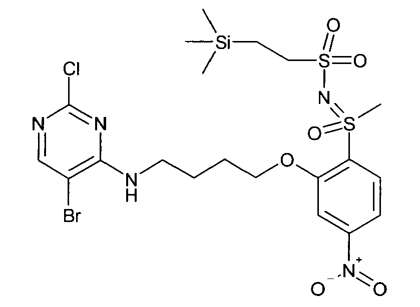

Ein Zweihalskolben mit Molekularsieb (3Å) wird unter Vakuum ausgeheizt und anschließend werden 300 mg (0,65 mmol) Verbindung 9.3 eingewogen. Es wird evakuiert und mit Argon gespült (3x). Man versetzt mit 15 ml Acetonitril und rührt 10 min bei Raumtemperatur. Anschließend werden unter Argon 150 mg (0,40 mmol) CuPF₆(MeCN)₄ zugegeben und 20 min bei Raumtemperatur gerührt. Das Reaktionsgemisch wird auf 0°C abgekühlt und man versetzt mit 300 mg (0,78 mmol) Ph-I=N-Ses Reagenz (H. Tye, C.L. Skinner, T.C. Kinahan, S. Cren Tetrahedron Lett. 2002, 43, 2749-2751). Das Eisbad wird entfernt und man rührt 90 min bei Raumtemperatur. Nach DC Kontrolle wird der Ansatz erneut auf 0°C abgekühlt und man versetzt mit 150 mg (0.39 mmol) Ph-I=N-Ses Reagenz und rührt erneut bei Raumtemperatur. Im Verlauf der weiteren 18 h wird dieser Vorgang 3 Mal wiederholt und insgesamt 616 mg (1,61 mmol) Ph-1=N-Ses Reagenz sowie 130 mg (0.35 mmol) CuPF₆(MeCN)₄ zugegeben Der Ansatz wird mit Essigester verdünnt und mit gesättigter NaCl-Lösung gewaschen. Die wässrige Phase wird erneut gegen Essigester extrahiert (2x). Die vereinten organischen Phasen werden getrocknet (Na₂SO₄), filtriert und eingeengt. Der verbleibende Rückstand wird chromatographisch gereinigt (Hexan / EtOAc 4: 1). Man erhält 280 mg (0,44 mmol, entsprechend 67 % der Theorie) des Produktes.

¹H-NMR (DMSO): 8.22 (s, 1 H), 8.10 (m, 1 H), 8.05 (m, 2H), 7.72 (t, 1 H), 4.39 (m, 2H), 3.65 (s, 3H), 3.42 (m, 2H), 2.90 (m, 2H), 1.82 (m, 4H), 0.91 (m, 2H), -0.02 (s, 9H).

MS : 642 (ES).

### e) Verbindung 9.5

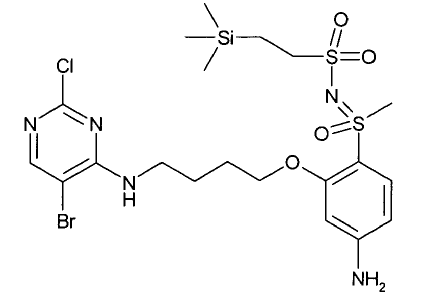

Eine Lösung von 280 mg (0,44 mmol) Verbindung 9.4 in 20 ml THF wird unter Argon bei Raumtemperatur mit 3,0 ml einer ca. 10%igen Lösung von Titan(III)Chlorid in 20-30%iger Salzsäure versetzt. Nach 20 min wird die Reaktionslösung erneut mit 0,5 ml der Titan(III)Chlorid-Lösung versetzt und weitere 4 Stunden gerührt. Man versetzt erneut mit 0,5 ml des Reduktionsmittels und rührt über Nacht. Nach DC Kontrolle wird mit 0,3 ml der Titan(III)Chlorid-Lösung versetzt. Nach 2 Stunden wird der Ansatz wird mit Essigester verdünnt und mit 1 N NaOH-Lösung basisch gestellt. Die Phasen werden getrennt und die wässrige Phase wird erneut gegen Essigester extrahiert. Die vereinten organischen Phasen werden getrocknet (Na₂SO₄), filtriert und eingeengt. Der verbleibende Rückstand wird chromatographisch gereinigt (DCM / EtOH 9:1). Man erhält 184 mg (0,30 mmol, entsprechend 69 % der Theorie) des Produktes.

¹H-NMR (DMSO): 8.22 (s, 1 H), 7.73 (t, 1 H), 7.42 (m, 1 H), 6.35 (m, 4H), 4.05 (m, 2H), 3.40 (m, 5H), 2.80 (m, 2H), 1.78 (m, 4H), 0.88 (m, 2H), - 0.02 (s, 9H).

MS : 612 (ES).

### 2. Herstellung des Endproduktes

Eine Lösung von 178 mg (0,29 mmol) Verbindung 9.5 in Acetonitril / Wasser / n-Butanol (9 ml / 1 ml / 3 ml) wird mittels Spritzenpumpe innerhalb von 4 Stunden zu einer refluxierenden Lösung von Acetonitril / Wasser / 4 N Lösung von Chlorwasserstoff in Dioxan (45 ml / 5 ml / 0,5 ml) gegeben. Nach 10 Tagen wird mit Wasser verdünnt und gegen Essigester extrahiert (2x). Die wässrige Phase wird mit NaHCO₃-Lösung neutral gestellt und erneut gegen Essigester extrahiert (2x). Die vereinten organischen Phasen werden getrocknet (Na₂SO₄), filtriert und eingeengt. Der erhaltene Rückstand wird chromatographisch gereinigt (DCM / EtOH 9:1). Man erhält 80 mg (0,14 mmol, entsprechend 48 % der Theorie) des Produktes.

¹H-NMR (DMSO): 9.98 (s, 1 H), 9.24 (m, 1 H), 8.10 (s, 1 H), 7.61 (m, 1 H), 7.55 (t, 1 H), 6.85 (m, 1 H), 4.53 (m, 2H), 3.53 (s, 3H), 3.40 (m, 2H), 2.80 (m, 2H), 1.91 (m, 4H), 0.91 (m, 2H), -0.02 (s, 9H).

MS: 576 (ES).

### Verfahrensvariante 3

### Beispiel 10: (RS)-N-(Ethoxycarbonyl)-S -methyl-S -[1⁵-3-pyridyl-2,4,9-triaza-1(2,4)-pyrimidina-3(1,3)-benzenacyclononaphan-3⁴-yl]sulfoximide

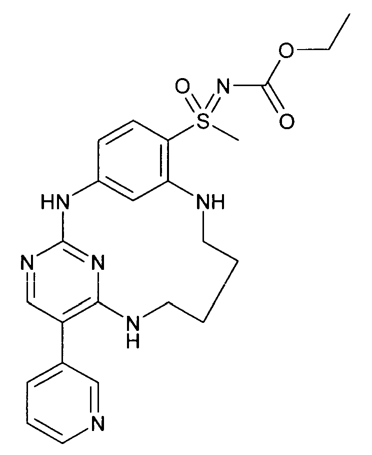

150 mg (0,28 mmol) (RS)-N-(Ethoxycarbonyl)-S-[1⁵-iod-2,4,9-triaza-1 (2,4)-pyrimidina-3(1,3)-benzenacyclononaphan-3⁴-yl]-S-methylsulfoximid (Beispiel 4), 52 mg (0,42 mmol), 3-Pyridinboronsäure und 122 mg (0,11 mmol) Palladiumtetrakistriphenylphosphin in 5 ml Dimethoxyethan werden unter Argon mit 1,6 ml einer 0,5 molaren Natriumhydroxid-Lösung versetzt. Der Ansatz wird mit Argon gespült und auf 90°C erwärmt. Nach 90 Minuten wird der Ansatz abgekühlt und in eine gesättigte NaCl-Lösung gegeben. Man extrahiert mit Essigester. Die vereinten organischen Phasen werden getrocknet (Na₂SO₄), filtriert und eingeengt. Der erhaltene Rückstand wird chromatographisch gereinigt.

MS: 482 (ES+)

### Assay 1

Aurora-C Kinase Assay

Rekombinantes Aurora-C Protein wurde in transient-transfizierten HEK293 Zellen exprimiert und anschließend gereinigt. Als Kinase-Substrat wurde das biotinylierte Peptid mit der Aminosäuresequenz biotin-FMRLRRLSTKYRT verwendet, das bei der Fa. Jerini AG in Berlin gekauft wurde.
Aurora-C [5 nM im Testansatz, Testvolumen 5 µl] wurde für 90 min bei 22°C in Anwesenheit verschiedener Konzentrationen an Testsubstanzen (0 µM, sowie 10 Messpunkten innerhalb des Bereiches 0,001 - 20 µM in Doppelwerten) in Assaypuffer [25 mM HEPES pH7,4, 0.5 mM MnCl₂, 0,1 mM Na ortho-Vanadat, 2,0 mM Dithiothreitol, 0.05% Bovines Serumalbumin (BSA), 0.01% Triton X-100, 3 µM Adenosintrisphosphat (ATP), 0,67 nCi/µl gama-P33-ATP, 2,0 µM Substratpeptid biotin-FMRLRRLSTKYRT, 1,0% Dimethylsulfoxid] inkubiert. Die Reaktion wurde durch Zugabe von 12.5 µl einer EDTA/Detektions-Lösung [16 mM EDTA, 40 mM ATP, 0.08% Triton X-100, 4 mg/ml PVT-Streptavidin-SPA-Beads (Fa. Amersham)] gestoppt. Nach 10 Minuten Inkubation wurden die SPA-Beads durch 10 minütige Zentrifugation bei 1000 x G pelletiert. Die Messung erfolgte in einem Topcount Szintillationsmessgerät der Firma PerkinElmer.
Die Messdaten wurden normalisiert auf 0% Inhibition (Enzymreaktion ohne Inhibitor) und 100% Inhibition (Enzymreaktion in Gegenwart von 0.1 µM Staurosporin (Fa. Sigma)). Die Bestimmung der IC50 Werte erfolgte mittels eines 4-Parameter Fits unter Benutzung firmeneigener Software.

### Assay 2

Aurora-A Kinase Assay

Rekombinantes Aurora-A Protein, exprimiert in Sf21 Insektenzellen, von der Firma Upstate gekauft. Als Kinase-Substrat wurde das biotinylierte Peptid mit der Aminosäuresequenz biotin-LNYNRRLSLGPMF verwendet, das bei der Fa. Jerini AG in Berlin gekauft wurde.
Aurora-A [15 nM im Testansatz, Testvolumen 5 µl] wurde für 90 min bei 22°C in Anwesenheit verschiedener Konzentrationen an Testsubstanzen (0 µM, sowie 10 Messpunkten innerhalb des Bereiches 0,001 - 20 µM in Doppelwerten) in Assaypuffer [25 mM HEPES pH7,4, 3 mM MnCl₂, 5 mM MnCl₂, 0,1 mM Na ortho-Vanadat, 2,0 mM Dithiothreitol, 0.05% Bovines Serumalbumin (BSA), 0.01% Triton X-100, 8 µM ATP, 4 nCi/µl gama-P33-ATP, 5,0 µM Substratpeptid biotin- LNYNRRLSLGPMF, 1,0% Dimethylsulfoxid] inkubiert. Die Reaktion wurde durch Zugabe von 12.5 µl einer EDTA/Detektions-Lösung [16 mM EDTA, 40 mM ATP, 0.08% Triton X-100, 4 mg/ml PVT-Streptavidin-SPA-Beads (Fa. Amersham)] gestoppt. Nach 10 Minuten Inkubation wurden die SPA-Beads durch 10 minütige Zentrifugation bei 1000 x G pelletiert. Die Messung erfolgte in einem Topcount Szintillationsmessgerät der Firma PerkinElmer.
Die Messdaten wurden normalisiert auf 0% Inhibition (Enzymreaktion ohne Inhibitor) und 100% Inhibition (Enzymreaktion in Gegenwart von 0.1 µM Staurosporin (Fa. Sigma)). Die Bestimmung der 1C50 Werte erfolgte mittels eines 4-Parameter Fits unter Benutzung firmeneigener Software.

### Assay 3

### CDK1/CycB Kinase Assay

Rekombinante CDK1- und CycB-GST-Fusionsproteine, gereinigt aus Bakulovirus-infizierten Insektenzellen (Sf9), wurden von ProQinase GmbH, Freiburg, gekauft. Das als Kinase-Substrat verwendet Histon IIIS ist über die Fa. Sigma käuflich zu erwerben.
CDK1/CycB (200 ng/Messpunkt) wurde für 10 min bei 22°C in Anwesenheit verschiedener Konzentrationen an Testsubstanzen (0 µM, sowie innerhalb des Bereiches 0,01 - 100 µM) in Assaypuffer [50 mM Tris/HCl pH8,0, 10 mM MgCl2, 0,1 mM Na ortho-Vanadat, 1,0 mM Dithiothreitol, 0,5 µM ATP, 10 µg/Messpunkt Histon IIIS, 0,2 µCi/Messpunkt 33P-gamma ATP, 0,05% NP40, 1,25% Dimethylsulfoxid] inkubiert. Die Reaktion wurde durch Zugabe von EDTA-Lösung (250 mM, pH8,0, 15 µl/Messpunkt) gestoppt.
Von jedem Reaktionsansatz wurden 15 µl auf P30 Filterstreifen (Fa. Wallac) aufgetragen, und nicht-eingebautes 33P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLexTM A, Fa. Wallac) bedeckt und für 1 Stunde bei 90°C eingebrannt. Die Menge an eingebautem 33P (Substratphosphorylierung) wurde durch Szintillationsmessung in einem Gamma-Strahlungsmessgerät (Wallac) bestimmt.

### Assay 4

### CDK2/CycE Kinase Assay

Rekombinante CDK2- und CycE-GST-Fusionsproteine, gereinigt aus Bakulovirus-infizierten Insektenzellen (Sf9), wurden von ProQinase GmbH, Freiburg, gekauft. Histon IIIS, das als Kinase-Substrat verwendet wurde, wurde bei der Fa. Sigma gekauft.
CDK2/CycE (50 ng/Messpunkt) wurde für 10 min bei 22°C in Anwesenheit verschiedener Konzentrationen an Testsubstanzen (0 µM, sowie innerhalb des Bereiches 0,01 - 100 µM) in Assaypuffer [50 mM Tris/HCl pH8,0, 10 mM MgCl₂, 0,1 mM Na ortho-Vanadat, 1,0 mM Dithiothreitol, 0,5 µM ATP, 10 µg/Messpunkt Histon IIIS, 0,2 µCi/Messpunkt ³³P-gamma ATP, 0,05% NP40, 1,25% Dimethylsulfoxid] inkubiert. Die Reaktion wurde durch Zugabe von EDTA-Lösung (250 mM, pH8,0, 15 µl/Messpunkt) gestoppt.
Von jedem Reaktionsansatz wurden 15 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt.

Nach dem Trocknen der Filterstreifen für 1 Stunde bei 70°C wurden die Filterstreifen mit Szintillator-Streifen (MeltiLex^{™} A, Fa. Wallac) bedeckt und für 1 Stunde bei 90°C eingebrannt. Die Menge an eingebautem ³³P (Substratphosphorylierung) wurde durch Szintillationsmessung in einem Gamma-Strahlungsmessgerät (Wallac) bestimmt.

### Assay 5

### Chk1 Kinase Assay

Rekombinantes Chk1 Protein wurde in Sf9 Insektenzellen exprimiert und anschließend gereinigt. Als Kinase-Substrat wurde das biotinylierte Peptid mit der Aminosäuresequenz biotin-ALKLVRTPSFVITAK verwendet, das bei der Fa. Biosynthan GmbH in Berlin gekauft wurde.
Chk1 [0.11 µg/ml im Testansatz, Testvolumen 5 µl] wurde für 60 min bei 22°C in Anwesenheit verschiedener Konzentrationen an Testsubstanzen (0 µM, sowie 10 Messpunkten innerhalb des Bereiches 0,001 - 20 µM in Doppelwerten) in Assaypuffer [50 mM HEPES pH7,5, 10 mM MgCl₂, 1,0 mM MgCl₂, 0,1 mM Na ortho-Vanadat, 1,0 mM Dithiothreitol, 1 Tablette / 2.5 ml Complete Proteaseinhibitor (Fa. Roche), 10 µM ATP, 1,0 µM Substratpeptid biotin-ALKLVRTPSFVITAK, 1,0% Dimethylsulfoxid] inkubiert. Die Reaktion wurde durch Zugabe von 5 µl einer EDTA/Detektions-Lösung [100 mM EDTA, 800 mM Kaliumfluorid, 0.2% BSA, 0,2 µM Streptavidin-XLent (Fa. CisBio), 9,6 nM antiphospho-Akt Antikörper (Fa. Cell Signalling Technology), 4 nM Protein-A-Eu(K) (Fa. CisBio)] gestoppt. Die Messung der Fluoreszenzemission bei 620 nm und 665 nm nach Anregung mit Licht er Wellenlänge 350 nm erfolgte in einem Rubystar HTRF-Messgerät der Firma BMG Labsystems.
Die Messdaten (Ratio aus Emission 665 geteilt durch Emission 620 multipliziert mit 10000) wurden normalisiert auf 0% Inhibition (Enzymreaktion ohne Inhibitor) und 100% Inhibition (alle Assaykomponenten außer Enzym). Die Bestimmung der IC50 Werte erfolgte mittels eines 4-Parameter Fits unter Benutzung firmeneigener Software.

### Assay 6

### c-kit Kinase Assay

Rekombinantes c-kit Protein wurde in E. coli exprimiert und anschließend gereinigt. Als Kinase-Substrat wurde das biotinyliertes Peptid mit der Aminosäuresequenz biotin-poly GluTyr verwendet, das bei der Fa. CisBio gekauft wurde.
C-kit [Testvolumen 5 µl] wurde für 30 min bei 22°C in Anwesenheit verschiedener Konzentrationen an Testsubstanzen (0 µM, sowie 10 Messpunkten innerhalb des Bereiches 0,001 - 20 µM in Doppelwerten) in Assaypuffer [50 mM HEPES pH7,0, 1,0 mM MgCl₂, 1,0 mM MgCl₂, 0,1 mM Na ortho-Vanadat, 1,0 mM Dithiothreitol, 0.001 % NP40, 10 µM ATP, 0,03 µM Substratpeptid biotin- poly GluTyr, 1,0% Dimethylsulfoxid] inkubiert. Die Reaktion wurde durch Zugabe von 5 µl einer EDTA/Detektions-Lösung [Lösung [50 mM HEPES pH7,5, 80 mM EDTA, 0.2% BSA, 0,1 µM Streptavidin-XLent (Fa. CisBio), 1 nM PT66-Eu (Fa. PerkinElmer)] gestoppt. Die Messung der Fluoreszenzemission bei 620 nm und 665 nm nach Anregung mit Licht er Wellenlänge 350 nm erfolgte in einem Rubystar HTRF-Messgerät der Firma BMG Labsystems.
Die Messdaten (Ratio aus Emission 665 geteilt durch Emission 620 multipliziert mit 10000) wurden normalisiert auf 0% Inhibition (Enzymreaktion ohne Inhibitor) und 100% Inhibition (alle Assaykomponenten außer Enzym). Die Bestimmung der IC50 Werte erfolgte mittels eines 4-Parameter Fits unter Benutzung firmeneigener Software.

### Assay 7

### KDR Kinase Assay

Rekombinantes KDR Protein wurde in E. coli exprimiert und anschließend gereinigt. Als Kinase-Substrat wurde das biotinyliertes Peptid mit der Aminosäuresequenz biotin-DFGLARDMYDKEYYSVG verwendet, das bei der Fa. Biosynthan gekauft wurde.
KDR [Testvolumen 5 µl] wurde für 45 min bei 22°C in Anwesenheit verschiedener Konzentrationen an Testsubstanzen (0 µM, sowie 10 Messpunkten innerhalb des Bereiches 0,001 - 20 µM in Doppelwerten) in Assaypuffer [50 mM HEPES pH7,0, 25,0 mM MgCl₂, 1,0 mM MgCl₂, 0,1 mM Na ortho-Vanadat, 1,0 mM Dithiothreitol, 0.001% NP40, 10 µM ATP, 0,03 µM Substratpeptid biotin- poly GluTyr, 1,0% Dimethylsulfoxid] inkubiert. Die Reaktion wurde durch Zugabe von 5 µl einer EDTA/Detektions-Lösung [50 mM HEPES pH7,5, 125 mM EDTA, 0.2% BSA, 0,1 µM Streptavidin-XLent (Fa. CisBio), 2 nM PT66-Eu (Fa. PerkinElmer)] gestoppt. Die Messung der Fluoreszenzemission bei 620 nm und 665 nm nach Anregung mit Licht er Wellenlänge 350 nm erfolgte in einem Rubystar HTRF-Messgerät der Firma BMG Labsystems.
Die Messdaten (Ratio aus Emission 665 geteilt durch Emission 620 multipliziert mit 10000) wurden normalisiert auf 0% Inhibition (Enzymreaktion ohne Inhibitor) und 100% Inhibition (alle Assaykomponenten außer Enzym). Die Bestimmung der IC50 Werte erfolgte mittels eines 4-Parameter Fits unter Benutzung firmeneigener Software.

### Assay 8

### Tie-2 Kinase Assay

Rekombinantes Tie-2 Protein wurde in Hi5 Insektenzellen exprimiert und anschließend gereinigt. Als Kinase-Substrat wurde das biotinyliertes Peptid mit der Aminosäuresequenz biotin-EPKDDAYPLYSDFG verwendet, das bei der Fa. Biosynthan gekauft wurde.
Tie-2 [Konzentration im Ansatz 5 ng/µl] wurde für 20 min bei 22°C in Anwesenheit von 100 µM ATP in Assaypuffer [50 mM HEPES pH7,0, 0,5 mM MgCl₂, 1,0 mM Dithiothreitol, 0.01 % NP40, 1 Tablette/2.5 ml Complete Proteaseinhibitor (Fa. Roche)] vorinkubiert. In Anwesenheit verschiedener Konzentrationen an Testsubstanzen (0 µM, sowie 10 Messpunkten innerhalb des Bereiches 0,001 - 20 µM in Doppelwerten) erfolgte im Anschluss die Enzymreaktion [0,5 ng/µl Tie-2 im Testansatz, Testvolumen 5 µl] für 20 min in Assaypuffer bei 10 µM ATP, 1,0 µM Substratpeptid biotin-EPKDDAYPLYSDFG, 1,0% Dimethylsulfoxid]. Die Reaktion wurde durch Zugabe von 5 µl einer EDTA/Detektions-Lösung [50 mM HEPES pH7,5, 89 mM EDTA, 0.28% BSA, 0,2 µM Streptavidin-XLent (Fa. CisBio), 2 nM PT66-Eu (Fa. PerkinElmer)] gestoppt. Die Messung der Fluoreszenzemission bei 620 nm und 665 nm nach Anregung mit Licht er Wellenlänge 350 nm erfolgte in einem Rubystar HTRF-Messgerät der Firma BMG Labsystems.
Die Messdaten (Ratio aus Emission 665 geteilt durch Emission 620 multipliziert mit 10000) wurden normalisiert auf 0% Inhibition (Enzymreaktion ohne Inhibitor) und 100% Inhibition (alle Assaykomponenten außer Enzym). Die Bestimmung der 1C50 Werte erfolgte mittels eines 4-Parameter Fits unter Benutzung firmeneigener Software.

### Beispiel 11

Die Verbindungen der Beispiele 1 bis 9 wurden in den verschiedenen Kinase-Assays hinsichtlich ihrer inhibitorischen Wirkung getestet.

Tabelle 1 zeigt, dass die erfindungsgemäßen Verbindungen Aurora im nanomolaren Bereich inhibieren, während die Inhibierung von CDKs schwächer ist. Weiterhin belegen die Beispiele, dass durch strukturelle Veränderungen die Inhibitionsprofile eingestellt werden können. So stellen beispielsweise die Verbindungen Nr. 4, Nr. 7 und Nr. 9 potente kombinierte Aurora, c-kit und VEGF-R2 (KDR) Inhibitoren dar.

**Tab. :1 IC₅₀-Werte**

| Beispiel Nr. | Aurora -C, [nM] | Aurora -A, [nM] | CDK1 [nM] | CDK2 [nM] | Chk1 [nM] | c-kit Kinase [nM] | KDR-Kinase [nM] | Tie-2-Kinase [nM] |
|---|---|---|---|---|---|---|---|---|
| 1 | 19 | 27 | 173 | 323 | 4643 | 29 | 61 | 2920 |
| 2 | 13 | 26 | 104 | 229 | 3626 | 46 | 198 | |
| 3 | 69 | 77 | 210 | 990 | 6839 | 64 | 289 | |
| 4 | 24 | 31 | 118 | 319 | 1850 | 23 | 9 | |
| 5 | 88 | 844 | > 1000 | > 1000 | > 20000 | 164 | 22 | |
| 6 | 19 | 21 | 70 | 71 | 3769 | 14 | 22 | 1413 |
| 7 | 34 | 44 | 452 | >1000 | > 20000 | 17 | 7 | 1699 |
| 8 | 283 | | 438 | > 1000 | > 20000 | 357 | 136 | 17110 |
| 9 | 95 | 652 | > 1000 | > 1000 | > 12500 | > 12500 | 60 | > 10000 |

## Patentansprüche

1. Verbindungen der allgemeinen Formel I, 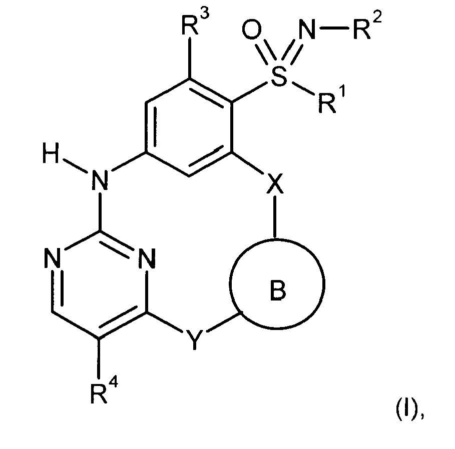 in der
B für eine Prop-1,3-ylen-, But-1,4-ylen-, Pent-1,5-ylen- oder Hex-1,6-ylen-gruppe, steht, die ein- oder mehrfach, gleich oder verschieden substituiert sein kann mit
(i) Hydroxy, -NR¹¹R¹², Cyano, Halogen, -CF₃, C₁-C₆-Alkoxy, -NR¹³-C(O)-C₁-C₃-Alkyl, -NR¹³-SO₂-C₁-C₃-Alkyl, -OCF₃ und/oder
(ii) einem oder mehreren C₁-C₆-Alkylresten, die gegebenenfalls mit Hydroxy, -NR¹¹R¹², Cyano, Halogen, -CF₃, C₁-C₆-Alkoxy, -NR¹³-C(O)-C₁-C₃-Alkyl, -NR¹³-SO₂-C₁-C₃-Alkyl oder -OCF₃ ein- oder mehrfach, gleich oder verschieden substituiert sind,
R¹ für
(i) einen gegebenenfalls mit Hydroxy, -NR¹¹R¹², Cyano, Halogen, C₁-C₆-Alkoxy, -CF₃ und/oder -OCF₃ ein- oder mehrfach, gleich oder verschieden substituierten C₁-C₆-Alkylrest oder
(ii) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierter C₃-C₇-Cycloalkylring oder
(iii) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierter C₆-Arylring oder
(iv) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierter Heteroarylring mit 5 oder 6 Ringatomen steht,
R² für R⁵, -SO₂-R⁶, -C(O)O-R⁶ , -C(O)-R⁶, -C(O)-NR¹¹R¹², -C(S)-NR¹¹R¹², -Si(R⁷R⁸R⁹), -R¹⁰-Si(R⁷R⁸R⁹) oder -SO₂-R¹⁰-Si(R⁷R⁸R⁹) steht,
R³ für
(i) Wasserstoff, Hydroxy, Halogen, Cyano, -CF₃, C₁-C₆-Alkoxy -OCF₃ oder -NR¹¹R¹² oder
(ii) einen gegebenenfalls mit Halogen, Hydroxy, C₁-C₆-Alkoxy oder der Gruppe -NR¹¹R¹² ein- oder mehrfach, gleich oder verschieden substituierten C₁-C₆-Alkylrest oder
(iii) eine gegebenenfalls mit Halogen, Hydroxy, C₁-C₆-Alkoxy oder der Gruppe -NR¹¹R¹² ein- oder mehrfach, gleich und/oder verschieden substituierte C₁-C₆-Alkoxygruppe steht oder
(iv) einen gegebenenfalls mit Halogen, Hydroxy, C₁-C₆-Alkoxy, der Gruppe -NR¹¹R¹² und/oder C₁-C₆-Alkyl ein- oder mehrfach, gleich oder verschieden substituierten C₃-C₇-Cycloalkylring steht,
R⁴ für
(i) Halogen, Cyano, Nitro, -NR¹¹R¹², -CF₃, C₁-C₆-Alkoxy oder -OCF₃ oder
(ii) einen gegebenenfalls mit Hydroxy, -NR¹¹R¹², Cyano, Halogen, C₁-C₆-Alkoxy, -CF₃ und/oder -OCF₃ ein- oder mehrfach, gleich oder verschieden substituierten C₁-C₆-Alkyl-, C₂-C₆-Alkenyl- oder C₂-C₆-Alkinylrest oder
(iii) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierten C₆-Arylring oder
(iv) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierten Heteroarylring mit 5 oder 6 Ringatomen steht,
X für -S-, -S(O)-, -NH- oder -O- steht,
Y für -NR¹³-, -S-, -S(O)-, oder -O- steht
wobei
R⁵ ein
(i) C₁-C₆-Alkylrest oder
(ii) C₃-C₆-Alkenylrest oder
(iii) C₃-C₆-Alkinylrest oder
(iv) C₆-Arylring oder
(v) Heteroarylring mit 5 oder 6 Ringatomen sein kann, die gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituiert sein können,
R⁶ ein
(i) C₁-C₆-Alkylrest oder
(ii) C₂-C₆-Alkenylrest oder
(iii) C₂-C₆-Alkinylrest oder
(iv) C₆-Arylring oder
(v) Heteroarylring mit 5 oder 6 Ringatomen sein kann, die gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituiert sein
können,
R⁷, R⁸ und R⁹ unabhängig voneinander
(i) ein C₁-C₆-Alkylrest, und/oder
(ii) ein C₆-Arylring sein können,
R¹⁰ für eine C₁-C₃-Alkylengruppe steht,
R¹¹ und R¹² unabhängig voneinander
(i) Wasserstoff und/oder
(ii) ein C₁-C₆-Alkylrest, und/oder
(iii) ein C₆-Arylring und/oder
(iv) ein Heteroarylring mit 5 oder 6 Ringatomen sein können, wobei (ii), (iii) und (iv) gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein können,
oder
R¹¹ und R¹² zusammen mit dem Stickstoffatom einen Heterocyclylring mit 3 bis 7 Ringatomen bilden, der gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert sein und ein weiteres Heteroatom enthalten kann, und
R¹³und R¹⁴ unabhängig voneinander für Wasserstoff oder einen C₁-C₆-Alkylrest stehen, der gegebenenfalls mit Hydroxy, -NH₂, Cyano, Halogen, -CF₃, C₁-C₆-Alkoxy und/oder -OCF₃ ein- oder mehrfach, gleich oder verschieden substituiert ist,
sowie deren Salze, Diastereomere und Enantiomere.

2. Verbindungen gemäß Anspruch 1,
in der
R¹ für einen gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituierten C₁-C₆-Alkylrest steht,
R² für R⁵, -SO₂-R⁶, -C(O)O-R⁶, -C(O)-R⁶, -C(O)-NR¹¹R¹² oder -SO₂-R¹⁰-Si(R⁶R⁷R⁸) steht,
R³ für Wasserstoff steht,
R⁴ für
(i) Halogen oder
(ii) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierten C₆-Arylring oder
(iii) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierten Heteroarylring mit 5 oder 6 Ringatomen steht,
X für -O- oder -NH- steht,
Y für -S- oder -NH- steht
wobei
R⁵, R⁶, R⁷, R⁸ und R⁹ unabhängig voneinander für einen C₁-C₆-Alkylrest stehen, die gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituiert sind,
R¹⁰ eine C₁-C₃-Alkylengruppe steht,
R¹¹ und R¹² unabhängig voneinander
(i) Wasserstoff und/oder
(ii) ein C₁-C₆-Alkylrest sein können, wobei (ii) gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert ist, und
R¹³und R¹⁴ unabhängig voneinander Wasserstoff oder ein C₁-C₆-Alkylrest sein können, der gegebenenfalls mit Hydroxy, -NH₂, Cyano, Halogen, -CF₃, C₁-C₆-Alkoxy und/oder -OCF₃ ein- oder mehrfach, gleich oder verschieden substituiert ist,
und B die in Anspruch 1 angegebene Bedeutung hat,
sowie deren Salze, Diastereomere und Enantiomere.

3. Verbindungen gemäß einem der Ansprüche 1 oder 2,
in der
B für eine Prop-1,3-ylen-, But-1,4-ylen- oder Pent-1,5-ylen-gruppe steht, die gegebenenfalls ein- oder mehrfach, gleich oder verschieden mit Hydroxy und/oder einem oder mehreren C₁-C₆-Alkylresten substituiert sein können, die gegebenenfalls mit -NR¹¹R¹², Cyano, Halogen, -CF₃, C₁-C₆-Alkoxy, -NR¹³-C(O)-C₁-C₃-Alkyl, -NR¹³-SO₂-C₁-C₃-Alkyl oder -OCF₃ ein- oder mehrfach, gleich oder verschieden substituiert sind,
sowie deren Salze, Diastereomere und Enantiomere.

4. Verbindungen gemäß einem der Ansprüche 1 bis 3,
B für eine Prop-1,3-ylen-, But-1,4-ylen- oder Pent-1,5-ylen-gruppe steht, die gegebenenfalls ein- oder mehrfach, gleich oder verschieden mit Hydroxy und/oder einem oder mehreren C₁-C₆-Alkylresten substituiert sein können, die gegebenenfalls mit -NR¹¹R¹², C₁-C₆-Alkoxy, -NR¹³-C(O)-C₁-C₃-Alkyl oder -NR¹³-SO₂-C₁-C₃-Alkyl ein- oder mehrfach, gleich oder verschieden substituiert sind,
sowie deren Salze, Diastereomere und Enantiomere.

5. Verbindungen gemäß einem der Ansprüche 1 bis 4,
in der
B eine But-1,4-ylen-gruppe bedeutet, die ein- oder mehrfach, gleich oder verschieden mit Hydroxy und/oder einem oder mehreren C₁-C₆-Alkylresten substituiert sein kann, die gegebenenfalls mit -NR¹¹R¹², Cyano, Halogen, -CF₃, C₁-C₆-Alkoxy, -NR¹³-C(O)-C₁-C₃-Alkyl, -NR¹³-SO₂-C₁-C₃-Alkyl oder -OCF₃ ein- oder mehrfach, gleich oder verschieden substituiert sind,
sowie deren Salze, Diastereomere und Enantiomere.

6. Verbindungen gemäß einem der Ansprüche 1 bis 5,
in der
B für eine But-1,4-ylen-gruppe steht, die ein- oder mehrfach, gleich oder verschieden mit Hydroxy und/oder einem oder mehreren C₁-C₆-Alkylresten substituiert sein kann, die gegebenenfalls mit -NR¹¹R¹², C₁-C₆-Alkoxy, -NR¹³-C(O)-C₁-C₃-Alkyl oder -NR¹³-SO₂-C₁-C₃-Alkyl ein- oder mehrfach, gleich oder verschieden substituiert sind,
sowie deren Salze, Diastereomere und Enantiomere.

7. Verbindungen gemäß einem der Ansprüche 1 bis 6,
in der
Y für -NH- oder -S- steht,
sowie deren Salze, Diastereomere und Enantiomere.

8. Verbindungen gemäß einem der Ansprüche 1 bis 7,
in der
X für -NH- oder -O- steht,
sowie deren Salze, Diastereomere und Enantiomere.

9. Verbindungen gemäß einem der Ansprüche 1 bis 7,
in der
R³ für
(i) Wasserstoff, Hydroxy, Halogen, C₁-C₆Alkoxy, -NR¹¹R¹² oder
(ii) einen gegebenenfalls mit Halogen, Hydroxy, C₁-C₆-Alkoxy oder der Gruppe -NR¹¹R¹² ein- oder mehrfach, gleich oder verschieden substituierter -C₁-C₆-Alkylrest oder
(iii) eine gegebenenfalls mit Halogen, Hydroxy, C₁-C₆-Alkoxy oder der Gruppe -NR¹¹R¹² ein- oder mehrfach, gleich und/oder verschieden substituierte C₁-C₆-Alkoxygruppe steht.

10. Verbindungen gemäß einem der Ansprüche 1 bis 9,
in der
R³ für Wasserstoff steht,
sowie deren Salze, Diastereomere und Enantiomere.

11. Verbindungen gemäß einem der Ansprüche 1 bis 10,
in der
R⁴ für
(i) Halogen oder -CF₃ oder
(ii) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierten C₆-Arylring oder
(iii) einen gegebenenfalls mit Hydroxy, -NR¹¹R¹², Cyano, Halogen, -CF₃, C₁-C₆-Alkoxy, -OCF₃ und/oder C₁-C₆-Alkyl ein- oder mehrfach, gleich oder verschieden substituierten Heteroarylring mit 5 oder 6 Ringatomen steht, wobei
R¹¹ und R ¹² unabhängig voneinander
(i) Wasserstoff und/oder
(ii) ein C₁-C₆-Alkylrest sein können, wobei (ii) gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert ist, und
R¹³und R¹⁴ unabhängig voneinander für Wasserstoff oder einen C₁-C₆-Alkylrest stehen, der gegebenenfalls mit Hydroxy, -NH₂, Cyano, Halogen, -CF₃, C₁-C₆-Alkoxy und/oder -OCF₃ ein- oder mehrfach, gleich oder verschieden substituiert ist.

12. Verbindungen gemäß einem der Ansprüche 1 bis 11,
in der
R⁴ für Halogen steht,
sowie deren Salze, Diastereomere und Enantiomere

13. Verbindungen gemäß einem der Ansprüche 1 bis 12,
in der
R² für R⁵, -SO₂-R⁶, -C(O)O-R⁶, -C(O)-R⁶, -C(O)-NR¹¹R¹² oder -SO₂-R¹⁰-Si(R⁷R⁸R⁹) steht,
wobei
R⁵, R⁶, R⁷, R⁸ und R⁹ unabhängig voneinander für einen C₁-C₆-Alkylrest steht, der gegebenenfalls mit Hydroxy, -NR¹¹R¹², Cyano, Halogen, -CF₃, C₁-C₆-Alkoxy und/oder -OCF₃ ein- oder mehrfach, gleich oder verschieden substituiert ist,
R¹⁰ für eine C₁-C₃-Alkylengruppe steht,
R¹¹ und R¹² unabhängig voneinander
(iii) Wasserstoff und/oder
(iv) ein C₁-C₆-Alkylrest sein können, wobei (ii) gegebenenfalls mit Hydroxy, -NR¹³R¹⁴, Cyano, Halogen, -CF₃, C₁-C₆-Alkoxy und/oder -OCF₃ ein- oder mehrfach, gleich oder verschieden substituiert ist, und
R¹³und R¹⁴ unabhängig voneinander Wasserstoff oder ein C₁-C₆-Alkylrest sein können, der gegebenenfalls mit Hydroxy, -NH₂, Cyano, Halogen, -CF₃ und/oder -OCF₃ ein- oder mehrfach, gleich oder verschieden substituiert ist.

14. Verbindungen gemäß einem der Ansprüche 1 bis 13,
in der
R² für -SO₂-R⁶, -C(O)O-R⁶, -C(O)-NR¹¹R¹² oder -SO₂-R¹⁰-Si(R⁷R⁸R⁹) steht, wobei
R⁶, R⁷, R⁸ und R⁹ unabhängig voneinander für C₁-C₅-Alkylreste stehen,
R¹⁰ für eine C₁-C₅-Alkylengruppe steht und
R¹¹ und R¹² unabhängig voneinander Wasserstoff und/oder ein C₁-C₆-Alkylrest sein können,
sowie deren Salze, Diastereomere und Enantiomere

15. Verbindungen gemäß einem der Ansprüche 1 bis 14,
in der
R⁵, R⁶, R⁷, R⁸ und R⁹ unabhängig voneinander für C₁-C₆-Alkylreste stehen,
sowie deren Salze, Diastereomere und Enantiomere.

16. Verbindungen gemäß einem der Ansprüche 1 bis 15,
in der
R¹⁰ für eine C₁-C₃-Alkylengruppe steht,
sowie deren Salze, Diastereomere und Enantiomere.

17. Verbindungen gemäß einem der Ansprüche 1 bis 16,
in der
R¹⁰ für Ethylen steht,
sowie deren Salze, Diastereomere und Enantiomere.

18. Verbindungen gemäß einem der Ansprüche 1 bis 17,
in der
R¹¹ und R¹² unabhängig voneinander Wasserstoff und/oder C₁-C₆-Alkylreste sein können,
sowie deren Salze, Diastereomere und Enantiomere.

19. Verbindungen der allgemeinen Formel I gemäß Anspruch 1,
in der
B für eine Prop-1,3-ylen-, But-1,4-ylen-, Pent-1,5-ylen- oder Hex-1,6-ylen-gruppe steht,
R¹ für einen C₁-C₅-Alkylrest steht,
R² für -SO₂-R⁵, -C(O)O-R⁶, -C(O)-NR¹¹R¹² oder -SO₂-R¹⁰-Si(R⁷R⁸R⁹) steht,
wobei
R⁵, R⁶, R⁷, R⁸ und R⁹ unabhängig voneinander für C₁-C₅-Alkylreste stehen,
R¹⁰ für eine C₁-C₅-Alkylengruppe steht,
R¹¹ und R¹² unabhängig voneinander Wasserstoff und/oder C₁-C₆-Alkylreste sein können,
R³ für Wasserstoff steht und
R⁴ für ein Halogen steht,
sowie deren Salze, Diastereomere und Enantiomere.

20. Verbindungen der allgemeinen Formel 1 gemäß Anspruch 1,
in der
B für eine But-1,4-ylen-gruppe steht,
R¹ für eine Methylgruppe steht,
R² für ein -SO₂-R⁶, -C(O)O-R⁶, -C(O)-NHR⁶ oder -SO₂-C₂H₄-Si(CH₃)₃ steht, wobei
R⁶ ein Ethyl- oder Propylrest sein kann,
R³ für Wasserstoff steht,
R⁴ für ein Halogen steht,
X für -O- oder -NH- steht, und
Y für -NH- steht.
sowie deren Salze, Diastereomere und Enantiomere.

21. Verbindungen der allgemeinen Formel I gemäß einem der vorstehenden Ansprüche 1 bis 19 zur Verwendung als Arzneimittel.

22. Verwendung von Verbindungen der allgemeinen Formel I gemäß einem der vorstehenden Ansprüche 1 bis 19 zur Herstellung eines Arzneimittels zur Behandlung von Krebs.

23. Verwendung von Verbindungen der allgemeinen Formel I gemäß Anspruch 22 zur Herstellung eines Arzneimittels zur Behandlung von kardiovaskulären Erkrankungen.

24. Verfahren zur Herstellung einer Verbindung gemäß einem der Ansprüche 1 bis 19, **gekennzeichnet durch** die Schritte:
a) Funktionalisierung der 4-Position von 2,4-Dichloropyrimidinderivaten der Formel **1a durch** Umsetzung mit Nucleophilen unter basischen Bedingungen, gegebenenfalls unter Verwendung einer Schutzgruppe für die Gruppe **X,** die nach Einführung von **1b** in die 4-Position von **1a** gegebenenfalls wieder abgespalten wird, 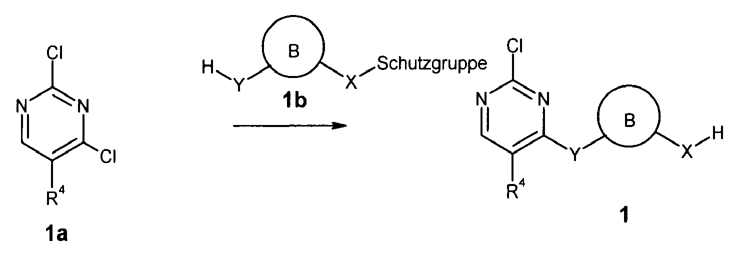
b) Oxidation einer Verbindung der Formel **2a** zum Sulfoxid der Formel **2b** 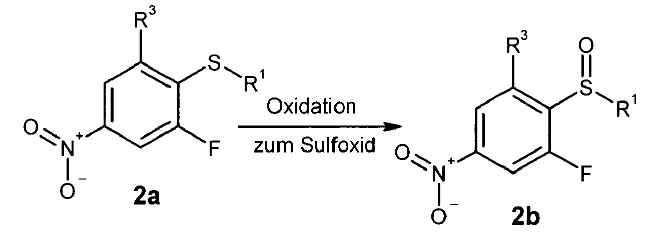
b₁) Umsetzung der Verbindung der Formel **2b** mit Natriumazid / Schwefelsäure zu einer Verbindung der Formel **2c** und N-Funktionalisierung des Sulfoximins zu einer Verbindung der Formel 2 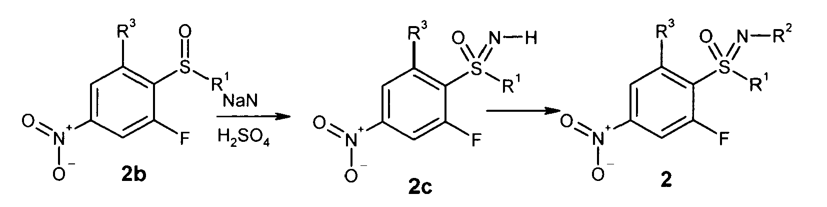 oder
b₂) direkte Umsetzung des Sulfoxides der Formel **2b** zu einer Verbindung der Formel **2,** 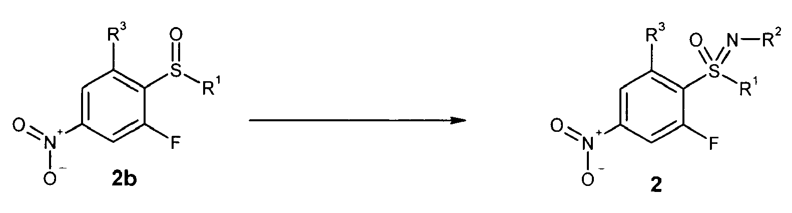
c) Umsetzung der Verbindung der Formel **1** aus Verfahrensschritt a) mit der Verbindung der Formel **2** aus Verfahrensschritt b) über eine nukleophile aromatischen Substitution zu einer Verbindung der Formel **3** 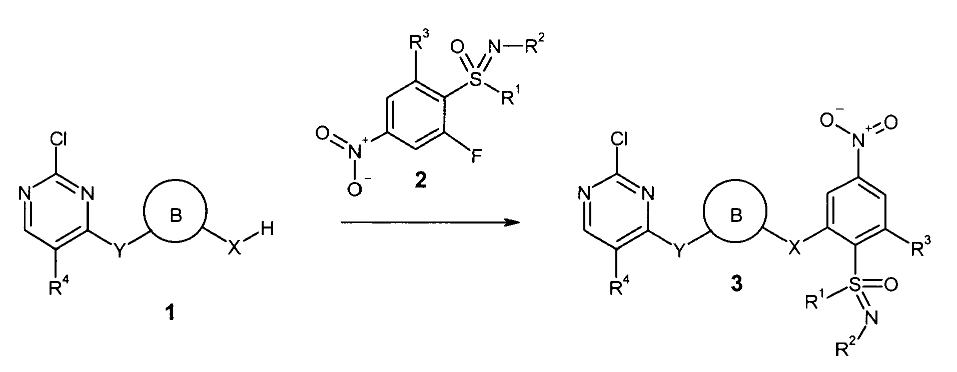
d) Reduktion der Verbindung der Formel **3** zu einer Verbindung der Formel **4** 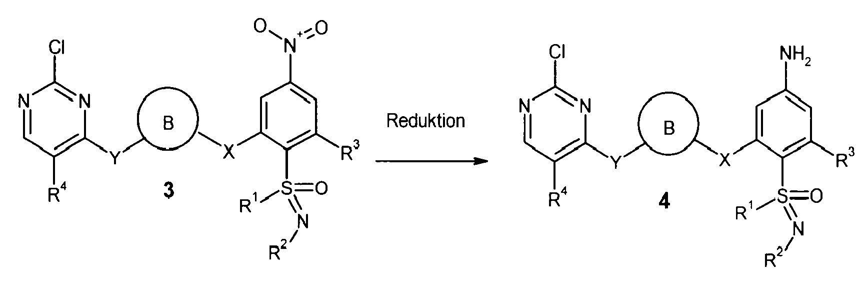
e) Cyclisierung der Verbindung der Formel **4** unter sauren Bedingungen zu Verbindungen der Formel **I**

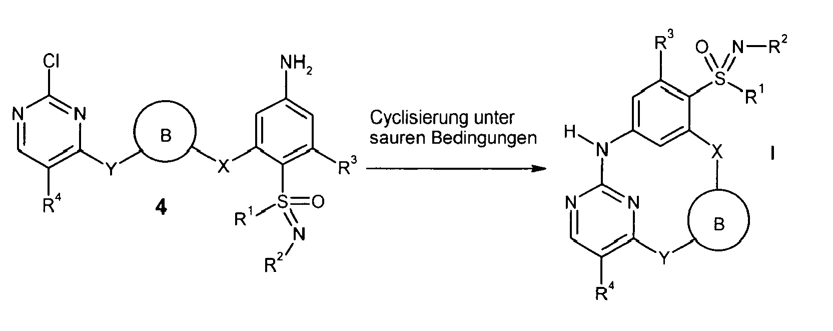

25. Verfahren zur Herstellung einer Verbindung gemäß einem der Ansprüche 1 bis 19, in denen X für -O- steht, **gekennzeichnet durch** die Schritte:
a) Umsetzung eines Alkohole der Formel **6** mit einem Phenol der Formel **7** unter Mitsunobu Bedingungen 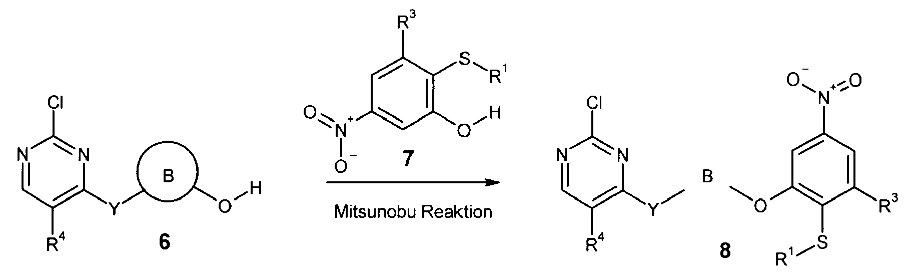
b)
(i) Oxidation des Thioethers der Formel 8 zum Sulfoxid und anschließend
(ii) Umsetzung zum Sulfoximin der Formel **9.** 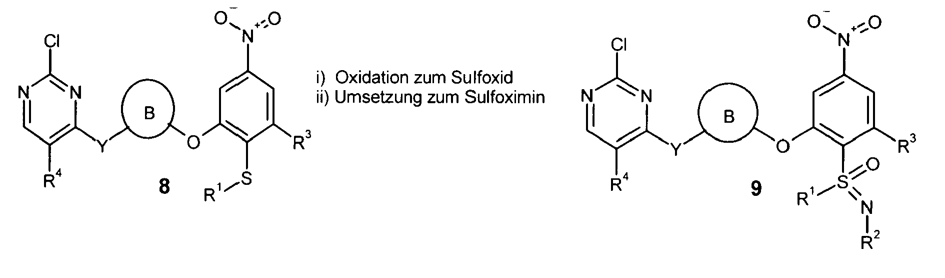
c)
(i) Reduktion der Verbindung der Formel **9** und
(ii) Cyclisierung unter sauren Bedingungen zu Verbindungen der Formel II. 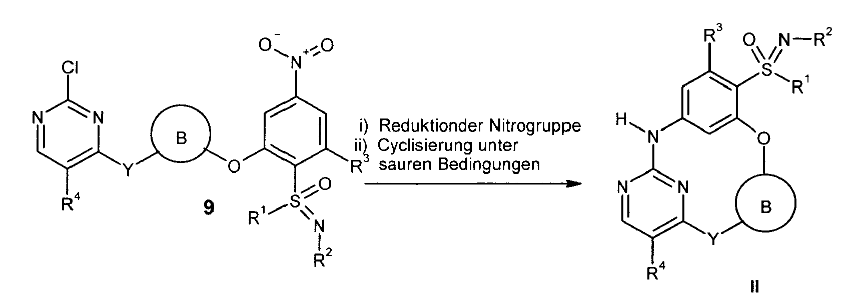
26. Pharmazeutische Formulierung enthaltend eine oder mehrere Verbindungen gemäß einem der Ansprüche 1 bis 19.
